# EUROPEAN PATENT SPECIFICATION

(11) **EP 0 460 178 B1**
(45) Date of publication and mention of the grant of the patent: **15.10.1997**
(21) Application number: 91901835.8
(22) Date of filing: 21.12.1990
(51) Int. Cl.: C12P 21/08, C12N 15/13, A61K 39/395, C07K 16/28, C12N 5/10, C12N 15/62

(54) **CD4 SPECIFIC RECOMBINANT ANTIBODY**
CD4-SPEZIFISCHER REKOMBINANTER ANTIKÖRPER
ANTICORPS DE RECOMBINAISON SPECIFIQUE DU CD4

(30) Priority: 21.12.1989 GB 8928874
(43) Date of publication of application: 11.12.1991
(73) Proprietor: ORTHO PHARMACEUTICAL CORPORATION, Raritan, NJ 08869-0602 (US)
(72) Inventor: JOLLIFFE, Linda Kay, Somerville, NJ 08876 (US); ZIVIN, Robert Allan, Lawrenceville, NJ 08648 (US); PULITO, Virginia Lee, Flemington, NJ 08822 (US); ADAIR, John Robert, High Wycombe, Buckinghamshire HP14 3RN (GB); ATHWAL, Diljeet Singh, London WC1 (GB)
(74) Representative: Mercer, Christopher Paul
(86) International application number: PCT/GB90/02015
(87) International publication number: WO 91/09966

(56) References cited:
- EP-A- 0 328 404
- EP-A- 0 365 209
- US-A- 4 695 459
- Proc. Natl. Acad. Sci. USA, vol., 86, December 1989, C. Queen et al.: "A humanized antibody that binds to the interleukin 2 receptor", see pages 10029- 10033, see the whole document and in particular right coll. page 10032, left col. and page 10033 left col.
- Nature, vol. 332, March 1988, L. Riechmann et al.: "Reshaping human antibodies for therapy", see pages 323-327: see in particular page 327, right col.
- Scientific American, Vol., October 1988 J.N. Weber et al.: "HIV Infection: The Cellular Picture", s. p. 81-p. 97
- Nature, Vol. 337, February 1989 D.J. Capon et al.: "Designing CD4 immunoadhesins for AIDS therapy ", s. p. 525-521

## Description

The present invention relates to CDR-grafted antibody molecules, to processes for their production using recombinant DNA technology and to their therapeutic uses.

In the present application, various prior art references are cited. These are referred to by a number given in square brackets []. The references are listed in numerical order at the end of the description.

In the present application, "Ig" is used to describe natural immunoglobulins. Natural immunoglobulins have been known for many years and comprise a generally Y-shaped molecule having an antigen-binding site towards the end of each upper arm. The remainder of the structure, and particularly the stem of the Y, mediates the effector functions associated with Igs. Various fragments of Igs, such as the Fab, (Fab')₂, Fv and Fc fragments, which can be derived by enzymatic cleavage, are also known.

Natural Igs comprise two heavy chains and two light chains, the N-terminal ends of each pair of heavy and light chains being associated and forming the antigen binding sites. The C-terminal ends of the heavy chains associate to form the Fc portion.

The residue designations for Ig light and heavy chains given in the present description and claims are in accordance with the numbering scheme developed by Kabat [1] and [2]. Thus, the residue designations do not always correspond directly with the linear numbering of the amino acid residues. The actual linear amino acid sequence may contains fewer or additional amino acid residues than in the strict Kabat numbering, thus showing that there have been insertions or deletions. These insertions or deletions may be present anywhere within the chains. The correct numbering of residues may be determined for a given Ig by alignment at regions of homology of the sequence of the Ig with a "standard" Kabat numbered sequence.

It was determined from a study of the amino acid sequences of a large number of Igs that the variable domains, which are located at the N-terminal ends of the chains, of both the heavy and the light chains contained three regions in which the amino acid sequence was hypervariable. These hypervariable regions are flanked on each side by regions which varied substantially less in sequence [1] and [2]. It was conjectured that the hypervariable regions are involved in antigen binding.

More recently, structural studies using X-ray crystallography and molecular modelling have defined three regions in the variable domains of each of the heavy and light chains which appear to be involved in antigen binding [47]. These three regions are generally referred to as the complementarity determining regions (CDRs). The CDRs are brought together by the remaining regions of the variable domains to form at least part of the antigen binding site. These remaining regions are generally referred to as the framework regions.

It will be appreciated that some workers in the art, and in particular Kabat [1] and [2], have referred to the hypervariable regions as being CDRs. For the sake of clarity, in this specification the term hypervariable region is used only to describe antigen binding regions determined by sequence analysis and the term CDR is used to describe antigen binding regions determined by structural analysis.

A comparison of the hypervariable regions, as determined by sequence analysis, and the CDRs, as determined by structural studies, shows that there is some, but not complete, correspondence between these regions.

In the present application, the term "antibody" is used to describe Igs or any fragments thereof, light chain or heavy chain monomers or dimers, and single chain antibodies, such as a single chain Fvs in which the heavy and light chain variable domains are joined by a peptide linker, whether natural or produced by recombinant DNA technology or otherwise, provided that the antibody includes at least one antigen binding site. The remainder of the antibody need not comprise only Ig-derived protein sequences. For instance, a gene may be constructed in which a DNA sequence encoding part of a human Ig chain is fused to a DNA sequence encoding the amino acid sequence of a polypeptide effector or reporter molecule. Thus, "antibody" encompasses hybrid antibodies (see below).

The abbreviation "MAb" is used to indicate a monoclonal antibody as produced by a hybridoma or derivative cell line.

The term "recombinant antibody" is used to describe an antibody produced by a process involving the use of recombinant DNA technology.

The term "chimeric antibody" is used to describe an antibody in which the variable domains as a whole are derived from an antibody from a first mammalian species and have been fused onto at least one constant domain from an antibody from a different mammalian species.

The term "hybrid antibody" is used to describe a protein comprising at least the antigen binding portion of an Ig attached by peptide linkage to at least part of another protein. It will be appreciated that certain skilled workers may also use the word "chimeric" to describe such constructs, but in the present specification such constructs are referred to as hybrid antibodies and the term chimeric antibodies is used in the sense defined above.

The term "CDR-grafted antibody" is used to describe an antibody having at least one, and preferably two or three, of its CDRs in one or both of the variable domains derived from an antibody from a first species, the remaining Ig-derived parts of the antibody being derived from one or more different antibodies. The variable domains may be made by use of recombinant DNA technology or by peptide synthesis.

"Expression vector" includes vectors which are capable of expressing DNA sequences contained therein, i.e. the coding sequences are operably linked to other sequences capable of effecting their expression. A useful, but not always necessary (i.e. insect cells), element of an effective expression vector is a marker encoding sequence, i.e. a sequence encoding a vector sequence which results in a phenotypic property (e.g. neomycin resistance, methionine sulfoximine resistance or tryptophan prototrophy) of the cells containing the protein which permits those cells to be readily identified. In sum, "expression vector" is given a functional definition and any DNA sequence which is capable of effecting expression of a specified contained DNA code is included in this term as it is applied to the specified sequence. As at present, such vectors are frequently in the form of plasmids. Thus "plasmid" and "expression vector" are often used interchangeably. However, the invention is intended to include such other forms of expression vectors which serve equivalent functions and which may, from time to time, become known in the art, including retroviruses, *in vitro* systems [48] and the like.

As stated previously, the DNA sequences will be expressed in host cells after the sequences have been operably linked to (i.e. positioned to ensure the functioning of) an expression control sequence. These expression vectors are typically replicable in the host organisms either as episomes or as an integral part of the host chromosomal DNA.

"Recombinant host cells" refers to cells which have been transformed with vectors constructed using recombinant DNA techniques. By virtue of this transformation, the host cell is able to produce the desired product in useful quantities, rather than in lesser amounts, or more commonly, in less than detectable amounts, as one would expect to be produced by the untransformed host. The antibody of the present invention may be produced by a recombinant host cell in quantities useful to carry out additional experimentation or in commercial quantities such as about a kilogram or more.

In descriptions of processes for isolation of antibodies from recombinant hosts, the terms "cell" and "cell culture" are used interchangeably to denote the source of antibody unless it is clearly specified otherwise. In other words, recovery of antibody from the "cells" may mean either from spun down whole cells, or from the cell culture containing both the medium and the suspended cells, or, additionally, as is possible in the case of myeloma cell lines, from ascites culture.

Natural Igs have been used in assay, diagnosis and, to a more limited extent, therapy. However, such uses, especially in therapy, have been hindered by the polyclonal nature of natural Igs. A significant step towards the realization of the potential of Igs as therapeutic agents was the discovery of techniques for the preparation of MAbs of defined specificity. MAbs are generally produced by fusions of rodent spleen cells with rodent myeloma cells, and thus are essentially rodent proteins. However, there are very few reports of the successful production of human MAbs.

A series of MAbs having specificities for antigens on T lymphocytes and subsets of T lymphocytes is described in EP-A-0 017 381, EP-A-0 018 794, EP-A-0 019 195, EP-A-0 025 722, EP-A-0 030 450, EP-A-0 030 814 and EP-A-0 033 578.

Since most available MAbs are entirely of rodent origin, they are naturally antigenic in humans and thus can give rise to an undesirable immune response, such as one response termed the Human Anti-Mouse Antibody (HAMA) response. Therefore, the use of rodent MAbs as therapeutic agents in humans is inherently limited by the fact that the human subject may mount an immunological response to the MAb and will either remove it entirely or at least reduce its effectiveness. Thus, in practice, MAbs of rodent origin are not generally recommended for use in patients for more than one or a few treatments, as a HAMA response may develop, rendering the MAb ineffective as well as giving rise to undesirable side reactions.

Proposals have therefore been made to render non-human MAbs less antigenic in humans. Such techniques can be generically termed "humanization" techniques. These techniques generally involve the use of recombinant DNA technology to manipulate DNA sequences encoding the polypeptide chains of the antibody molecule.

In recent years advances in molecular biology based on production of a wide range of heterologous polypeptides by transformation of host cells with heterologous DNA sequences which code for the production of the desired products.

EP-A-0 088 994 (Schering Corporation) proposes the construction of recombinant DNA vectors comprising a ds DNA sequence which codes for the variable domain of a light or a heavy chain of an Ig specific for a predetermined ligand. The ds DNA sequence is provided with initiation and termination codons at its 5'- and 3'-termini respectively but lacks any nucleotides coding for amino acids superfluous to the variable domain. The ds DNA sequence is used to transform bacterial cells. The application does not contemplate variations in the sequence of the variable domain.

EP-A-0 102 634 (Takeda Chemical Industries Limited) describes the cloning and expression in bacterial host organisms of genes coding for the whole or a part of a human IgE heavy chain polypeptide, but does not contemplate variations in the sequence of the polypeptide.

EP-A-0 125 023 (Genentech Inc.) proposes the use of recombinant DNA techniques in bacterial cells to produce Igs which are analagous to those normally found in vertebrate systems and to take advantage of the gene modification techniques proposed therein to construct chimeric antibodies or other modified forms of antibody.

It is believed that the proposals set out in the above Genentech application did not lead to the expression of any significant quantities of Ig polypeptide chains, nor to the production of Ig activity, nor to the secretion and assembly of the chains into the desired chimeric antibodies.

The recent emergence of techniques allowing the stable introduction of Ig gene DNA into mammalian cells [3] to [5] has opened up the possibility of using *in vitro* mutagenesis and DNA transfection to construct recombinant antibodies possessing novel properties.

However, it is known that the function of an antibody molecule is dependent on its three dimensional structure, which in turn is dependent on its primary amino acid sequence. Thus, changing the amino acid sequence of an antibody may adversely affect its activity. Moreover, a change in the DNA sequence coding for the antibody may affect the ability of the cell containing the DNA sequence to express, secrete or assemble the antibody.

It is therefore not at all clear that it will be possible to produce functional altered antibodies by recombinant DNA techniques. However, colleagues of the present inventors have devised a process whereby hybrid antibodies in which both parts of the protein are functional can be secreted. This process is disclosed in International Patent Application No. PCT/GB85/00392. However, the above PCT application only shows the production of hybrid antibodies in which complete variable domains are coded for by the first part of the DNA sequence. It does not show hybrid antibodies in which the sequence of the variable domain has been altered.

EP-A-0 239 400 describes a process in which the CDRs of a mouse MAb have been grafted onto the framework regions of the variable domains of a human Ig by site directed mutagenesis using long oligonucleotides. The inventors allude to the possibility of altering the natural amino acid sequence of the framework regions as well.

The earliest work on altering MAbs by CDR-grafting was carried out on MAbs recognizing synthetic antigens, such as the NP or NIP antigens. However, examples in which a mouse MAb recognizing lysozyme and a rat MAb recognizing an antigen on human T-cells respectively were humanized by CDR-grafting have been described [6] and [7].

Reference [7] shows that transfer of the CDRs alone (as defined in that paper) was not sufficient to provide satisfactory antigen binding activity in the CDR-grafted product. Reference [7] shows that it was necessary to convert a serine residue at position 27 of the human sequence to the corresponding rat phenylalanine residue to obtain a CDR-grafted product having satisfactory antigen binding activity. This residue at position 27 of the heavy chain is within the structural loop adjacent to CDR1. A further construct which additionally contained a human serine to rat tyrosine change at position 30 of the heavy chain did not have a significantly altered binding activity over the CDR-grafted antibody with the serine to phenylalanine change at position 27 alone. These results indicate that, for CDR-grafted antibodies which recognize more complex antigens, changes to residues of the human sequence outside the CDR regions, in particular in the loop adjacent to CDR1, may be necessary to obtain effective antigen binding activity.

Techniques have also recently been described for altering an anti-TAC monoclonal antibody by CDR-grafting. Human framework regions were chosen to maximize homology with the anti-TAC antibody sequence, while several additional amino acids outside the CDRs were retained. The anti-TAC antibody so altered has an affinity for the p55 chain of human interleukin-2 of about one third that of murine anti-TAC [8].

PCT/US89/05857 also describes CDR-grafted antibodies which are specific for the p55 TAC protein of the IL-2 receptor. It is therein stated that the CDR-grafted antibody may require that 3 or more amino acid residues from the donor Ig in addition to the CDRs, usually at least one of which is immediately adjacent to a CDR in the donor Ig, be changed to correspond to that of the donor antibody in order to obtain antigen binding activity.

It is therefore readily apparent that it is not a simple matter to produce a CDR-grafted antibody. It is often not sufficient merely to graft the CDRs from a donor Ig onto the framework regions from an acceptor Ig. It may also be necessary to alter residues in the framework regions of the acceptor antibody in order to obtain binding activity. However, it is not possible to predict, on the basis of the available prior art, which, if any, framework residues will need to be altered.

EP-A-0 018 794 describes a murine MAb which recognises an antigen characteristic of human helper T cells. A particular example of such an MAb is described in the application and is designated OKT4. The antigen it recognises is generally referred to as the CD4 antigen. The MAb is commercially available from Ortho Diagnostic Systems Inc. of Raritan, New Jersey, USA. Also available from the same supplier is a murine MAb known as OKT4A. This recognises a different epitope on the CD4 antigen from the one recognised by OKT4.

Transplantation experiments in primates have indicated that both OKT4 and OKT4A can extend graft survival and may be useful as an immunomodulator in humans. Experience from the treatment of renal transplant patients with the murine MAb OKT3 has shown that sometimes a population of patients develops neutralizing antibodies to OKT3. This immune response precludes repeat administration. To diminish the anticipated immune response to murine anti-CD4 MAbs, it would be desirable to produce a CDR-grafted version of OKT4A having murine CDRs and human framework and other Ig derived regions.

EP-A-0 365 209 (Becton Dickinson) describes the nucleotide and amino acid sequence of the mouse monoclonal antibody anti-Leu 3a which recognises the CD4 antigen. The application also describes chimeric and mosaic variants thereof.

However, as described above, the simple approach to constructing a CDR-grafted antibody does not always result in an antibody which effectively binds the antigen. The exact residues which comprise the CDRs are difficult to define and do not necessarily correspond to all the residues in the hypervariable regions. There may also be critical framework residues which are important in positioning the CDRs for interaction with antigen or which are involved in interactions between the heavy and light chains. It may be necessary to alter certain framework residues so that they correspond to the murine residues at these positions, rendering the CDR-grafted antibody less "human" in character.

Despite the problems which are inherent in attempting to produce a specific CDR-grafted antibody, the present inventors have succeeded in producing a CDR-grafted antibody based on human framework regions and having an antigen binding site which is derived from the murine MAb OKT4A.

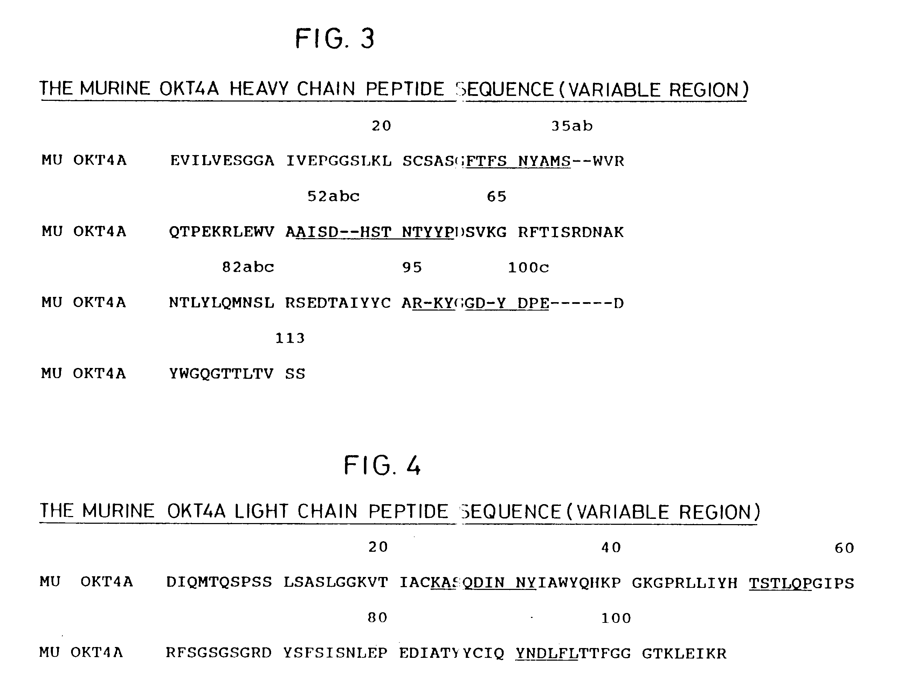

Therefore, according to the present invention, there is provided an antibody molecule capable of binding to the CD4 antigen comprising a composite heavy chain and a complementary light chain wherein, in the variable domain of said composite heavy chain, the framework regions are predominantly derived from a human antibody (acceptor) and at least residues 23, 24, 26 to 35, 49 to 65 and 95 to 102 (according to the Kabat numbering system) correspond to the equivalent residues in the mouse monoclonal antibody OKT4A (donor) as shown in Figure 3 of the accompanying drawings.

It is preferred that residues 6 and 48 in the composite heavy chain additionally correspond to the donor antibody in equivalent residue positions. If desired, residues 71, 73 and 79 can also so correspond.

To further optimise affinity, any one or any combination of residues 57, 58, 60, 88 and 91 may correspond to the equivalent residue in the donor antibody.

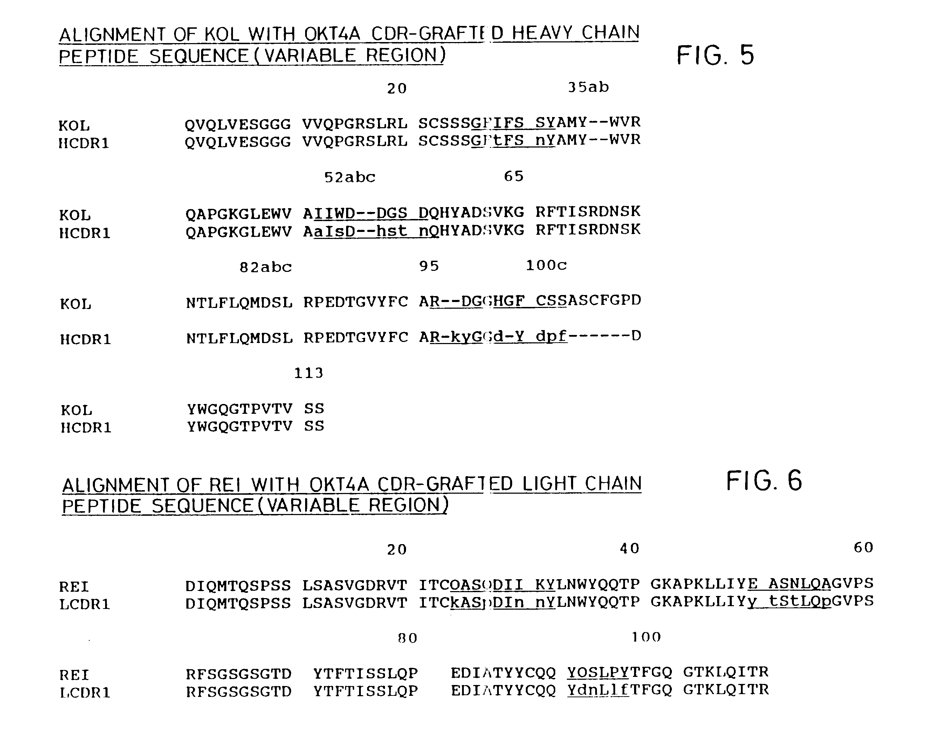

The heavy chain is preferably derived from the human KOL heavy chain as shown in Figure 5 of the accompanying drawings. However, it may also be derived from the human NEWM or EU heavy chain as shown in Kabat [1].

According to a second aspect of the present invention, there is provided an antibody molecule capable of binding to the CD4 antigen comprising a composite light chain and a complementary heavy chain wherein, in the variable domain of said composite light chain, the framework regions are predominantly derived from a human antibody (acceptor) and at least residues 24 to 34, 49 to 56 and 89 to 97 (according to the Kabat numbering system) correspond to the equievalent residues in the mouse monoclonal antibody OKT4A (donor) as shown in Figure 4 of the accompanying drawings.

To further optimise affinity, it is preferable to ensure that in the composite light chain residue 89 corresponds to the equivalent residue in the donor antibody. It may also be desirable to select equivalent donor residues that form salt bridges.

The light chain is preferably derived from the human REI light chain as shown in Figure 6 of the accompanying drawings. However, it may also be derived from the human EU light chain as shown in Kabat [1].

Preferably, the antibody of the first aspect of the present invention comprises as the complementary light chain a composite light chain as defined for the second aspect of the invention.

In the preferred case, it is advantageous that minimal alteration is made to the light chain.

It will be appreciated that in some cases, for both heavy and light chains, the donor and acceptor residues may be identical at a particular position and thus no change of acceptor framework residue will be required.

It will also be appreciated that in order to retain as far as possible the human nature of the antibody, as few residue changes as possible should be made. It is envisaged that in many cases, it will not be necessary to change more than the CDRs and a small number of framework residues. Only in exceptional cases will it be necessary to change a larger number of framework residues.

Preferably, the CDR-grafted antibody is a complete Ig, for example of isotype IgG₁ or IgG₄.

If desired, one or more residues in the constant domains of the Ig may be altered in order to alter the effector functions of the constant domains.

Preferably, the antibody of the invention has an affinity for the CD4 antigen of between 10⁵.M⁻¹ and 10¹².M⁻¹, more preferably at least 10⁸.M¹. and most preferably the affinity is similar to that of MAb OKT4 or OKT4A.

Advantageously, the CDR-grafted antibody of the present invention is produced by use of recombinant DNA technology.

According to a third aspect of the present invention, there is provided a method for producing an antibody according to the first or second aspect of the present invention, which method comprises: providing a first DNA sequence, encoding a composite heavy chain as defined above or a composite light chain as defined above, under the control of suitable upstream and downstream elements; transforming a host cell with the first DNA sequence; and culturing the transformed host cell so that an antibody according to the first or second aspect of the invention is produced.

Preferably, the method further comprises: providing a second DNA sequence, encoding a second antibody chain complementary to the first chain, under the control of suitable upstream and downstream elements; and transforming the host cell with both the first and second DNA sequences.

Advantageously, the second DNA sequence also encodes a composite antibody chain as defined above.

The first and second DNA sequences may be present on the same vector. In this case, the sequences may be under the control of the same or different upstream and/or downstream elements.

Alternatively, the first and second DNA sequences may be present on different vectors.

According to a fourth aspect of the present invention, there is provided a nucleotide sequence which encodes a composite antibody chain as defined above.

It is envisaged that the antibodies of the present invention will be of particular use in therapy, in particular in treating graft rejections or in treating helper T cell disorders.

The antibodies of the present invention may be produced by a variety of techniques, with expression in transfected cells, such as yeast, insect, CHO or myeloma cells, being preferred. Most preferably, the host cell is a CHO host cell.

To design the antibody of the present invention, it is first necessary to ascertain the variable domain sequence of the mouse monoclonal antibody OKT4A. The variable domain sequences (V_{H} and V_{L}) may be determined from neavy and light chain cDNA, synthesized from the respective mRNA by techniques generally known to the art. The hypervariable regions may then be determined using the Kabat method [2]. The CDRs may be determined by structural analysis using X-ray crystallography or molecular modelling techniques. A composite CDR may then be defined as containing all the residues in one CDR and all the residues in the corresponding hypervariable region. These composite CDRs along with certain select residues from the framework region are preferably transferred as the "antigen binding sites", while the remainder of the antibody, such as the heavy and light chain constant domains and remaining framework regions, are based on human antibodies of different classes. Constant domains may be selected to have desired effector functions appropriate to the intended use of the antibody so constructed. For example, human IgG isotypes, IgG₁ and IgG₃ are effective for complement fixation and cell mediated lysis. For other purposes other isotypes, such as IgG₂ and IgG₄, or other classes, such as IgM and IgE, may be more suitable.

For human therapy, it is particularly desirable to use human isotypes, to minimize antiglobulin responses during therapy. Human constant domain DNA sequences, preferably in conjunction with their variable domain framework bases can be prepared in accordance with well-known procedures. An example of this is CAMPATH 1H available from Burroughs Wellcome Ltd.

In accordance with preferred embodiments of the present invention, certain CDR-grafted antibodies are provided which contain select alterations to the human-like framework region (in other words, outside of the CDRs of the variable domains) resultinq in a CDR-qrafted antibody with satisfactory binding affinity. Such binding affinity is preferably from about 10⁵.M⁻¹ to about 10¹².M⁻¹ and is more preferably at least about 10⁸.M⁻¹. Most preferably the binding affinity is about equal to that of murine MAb OKT4A.

In constructing the CDR-grafted antibodies of the present invention, the V_{H} and/or V_{L} gene segments may be altered by mutagenesis. One skilled in the art will also understand that various other nucleotides coding for amino acid residues or sequences contained in the Fc portion or other areas of the antibody may be altered in like manner (see, for example, PCT/US89/00297).

Exemplary techniques include the addition, deletion or nonconservative substitution of a limited number of various nucleotides or the conservative substitution of many nucleotides, provided that the proper reading frame is maintained.

Substitutions, deletions, insertions or any subcombination may be used to arrive at a final construct. Since there are 64 possible codon sequences but only twenty known amino acids, the genetic code is degenerate in the sense that different codons may yield the same amino acid. However, the code is precise for each amino acid. Thus there is at least one codon for each amino acid, i.e. each codon yields a single amino acid and no other. It will be apparent that during translation, the proper reading frame must be maintained in order to obtain the proper amino acid sequence in the polypeptide ultimately produced.

Techniques for additions, deletions or substitutions at predetermined amino acid sites having a known sequence are well known. Exemplary techniques include oligonucleotide-mediated site-directed mutagenesis and the polymerase chain reaction.

Oligonucleotide site-directed mutagenesis in essence involves hybridizing an oligonucleotide coding for a desired mutation with a single strand of DNA containing the region to be mutated and using the single strand as a template for extension of the oligonucleotide to produce a strand containing the mutation. This technique, in various forms, is described in references [9] to [12].

Polymerase chain reaction (PCR) in essence involves exponentially amplifying DNA *in vitro* using sequence specific oligonucleotides. The oligonucleotides can incorporate sequence alterations if desired. The polymerase chain reaction technique is described in reference [13]. Examples of mutagenesis using PCR are described in references [14] to [17].

The nucleotide sequences of the present invention, capable of ultimately expressing the desired CDR-grafted antibodies, can be formed from a varietv of different polynucleotides (genomic DNA, cDNA, RNA or synthetic oligonucleotides). At present, it is preferred that the polynucleotide sequence comprises a fusion of cDNA and genomic DNA. The polynucleotide sequence may encode various Ig components (e.g. V, J, D, and C domains). They may be constructed by a variety of different techniques. Joining appropriate genomic and cDNA sequences is presently the most common method of production, but cDNA sequences may also be utilized (see EP-A-0 239 400 and [7]).

Certain suitable expression vectors and host cells are described in US-A-4 816 567.

The vectors and methods disclosed herein are suitable for use in host cells over a wide range of prokaryotic and eukaryotic organisms.

In general, of course, prokaryotes are preferred for cloning of DNA sequences for constructing the vectors useful in the invention. For example, *E. coli* DH5α is particularly useful. This example is, of course, intended to be illustrative rather than limiting.

Prokaryotes may also be used for expression. The aforementioned *E*. *Coli* strains, *bacilli* such as *Bacillus subtilus,* and other enterobacteriaceae, such as *Salmonella typhimurium* or *Serratia marcesans,* and various *Pseudomonas* species may be used.

In general, plasmid vectors containing replicon and control sequences which are derived from species compatible with the host cell are used in connection with these hosts. The vector ordinarily carries a replication site as well as marking sequences which are capable of providing phenotypic selection in transformed cells. For example, *E*. *Coli* is typically transformed using one of the many derivatives of pBR322, a plasmid derived from an E. *Coli* species [18]. pBR322 contains genes for ampicillin and tetracycline resistance and thus provides easy means for identifying transformed cells. The pBR322 plasmid, its descendents or other microbial plasmids may also contain, or be modified to contain, promoters which can be used by the microbial organism for the expression of recombinant proteins. Those promoters commonly used in recombinant DNA construction include lactose promoter systems [19] to [21] and tryptophan (trp) promoter systems [22] and EP-A-0 036 776. While these are the most commonly used, other microbial promoters have been discovered and utilized, and details concerning their nucleotide sequences have been published, enabling a skilled worker to ligate them functionally into plasmid vectors [23].

In addition to prokaryotes, eukaryotic microbes, such as yeast cultures, may also be used. *Saccharomyces cerevisiae,* or common baker's yeast, is the most commonly used among eukaryotic microorganisms, although a number of other strains are commonly available. For expression in *Saccharomyces,* the plasmid YRp7, for example, [24] to [26] is commonly used. This plasmid already contains the trpl gene which provides a selection marker for a mutant strain of yeast lacking the ability to grow in tryptophan, for example ATCC No. 44076 or PEP4-1 [27]. The presence of the trpl lesion as a characteristic of the yeast host cell genome then provides an effective environment for detecting transformation by growth in the absence of tryptophan.

Suitable promoting sequences in yeast vectors include the promoters for 3-phosphoglycerate kinase [28] or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase and glucokinase [29] and [30]. In constructing suitable expression plasmids, the termination sequences associated with these genes are also ligated into the expression vector 3' of the sequence desired to be expressed to provide polyadenylation of the mRNA and termination. Other promoters, which have the additional advantage of transcription controlled by growth conditions are the promoter regions for alcohol dehydrogenase 2, isocytochrome C, acid phosphatase, degradative enzymes associated with nitrogen metabolism and the aforementioned glyceraldehyde-3-phosphate dehydrogenase, an enzyme responsible for maltose and galactose utilization [30]. Any plasmid vector containing a yeast compatible promoter, origin of replication and termination sequences is suitable.

In addition to microorganisms, cultures of cells derived from multicellular organisms may also be used as hosts. In principle, any such cell culture is workable, whether from a vertebrate or an invertebrate organism. However, to date, interest has been greatest in vertebrate cells, and propogation of vertebrate cells in culture (tissue culture) has become a routine procedure in recent years [31]. Examples of such useful host cell lines are VERO, HeLa, Chinese hamster ovary (CHO), W138, BHK, COS-7, MDCK and myeloma cell lines. Expression vectors for such cells may include (if necessary) an appropriate origin of replication, as well as a promoter located in front of the gene to be expressed, along with any necessary ribosome binding sites, RNA splice sites, polyadenylation sites and transcriptional terminator sequences.

For use in mammalian cells, the control functions on the expression vectors are often provided by viral material. For example, commonly used promoters are derived from human Cytomegalovirus (HCMV), Polyoma virus, Adenovirus 2 and, most frequently, Simian Virus 40 (SV40). The early and late promoters of SV40 virus are particularly useful because both are obtained easily from the virus as a fragment which also contains the SV40 viral origin of replication [32]. Further, it is also possible, and often desirable, to utilize promoter or control sequences normally associated with the desired gene sequence, provided such control sequences are compatible with the host cell system.

An origin of replication may be prov-ided either by construction of the vector to include an exogenous origin, such as may be derived from SV40 or other viral (e.g. Polyoma virus, Adeno virus, VSV or BPV) source, or may be provided by the host cell chromosomal replication mechanism. If the vector is integrated into the host cell chromosome, the latter is often sufficient.

The vectors containing the DNA segments of interest (e.g. the heavy and light chain encoding sequences and expression control sequences) can be transferred into the host cell by well-known methods, which vary depending on the type of cellular host. For example, calcium chloride transfection is commonly utilized for prokaryotic cells, whereas calcium phosphate treatment, lipofection or electroporation may be used for other cellular hosts [33].

Once expressed, the CDR-grafted antibodies of the present invention can be purified according to standard procedures of the art, including ammonium sulfate precipitation, affinity columns, column chromatography and gel electrophoresis [34]. Binding affinities of the constructs so expressed may be ascertained by techniques known to the art, as more fully exemplified in the example section of this specification.

Substantially pure CDR-grafted antibodies of at least 90 to 95% homogeneity are preferred, and 98 to 99% or more homogeneity is most preferred for pharmaceutical uses. Once purified, partially or to homogeneity as desired, the CDR-grafted antibodies may then be used diagnostically or therapeutically (including extracorporeally) or in developing and performing assay procedures, immunofluorescent stainings and the like [35].

The CDR-grafted antibodies of the present invention will typically find use in treating T-cell mediated disorders. For example, typical disease states suitable for treatment include graft versus host disease and transplant rejection in patients undergoing an organ, such as heart, lung, kidney or liver, transplant, Other diseases include autoimmune diseases, such as Type I diabetes, multiple sclerosis, rheumatoid arthritis, systemic lupus erythematosus and myasthenia gravis.

T cells are clonal expansions from single cells expressing only one T cell antigen receptor capable of recognizing a peptide bound to a specific HLA molecule on specialized antigen presenting cells, such as a macrophages, and on other tissues. The activation of these T cells can be blocked by antibodies recognizing the T cell receptor complex or the peptide-HLA complex. OKT3 recognizes the CD3 molecule which is comprised of several subunits physically complexed with the T cell receptor. Several other molecules on the T cell, including the CD4 and CD8 molecules, are also involved in T cell activation by binding to the HLA molecules at sites that are distinct from the T cell receptor binding site.

CD4 is found on the subpopulation of T cells with T cell receptors that recognize HLA class II molecules. Therefore, one approach to immunosuppression involves the use of monoclonal antibodies, such as OKT4 or OKT4A that are immunosuppressive because they inhibit the interaction of the CD4 molecule with the HLA class II molecule. Antibody binding to CD4 can result in immunosuppression by a number of mechanisms including the inhibition of a normal activation signal, the triggering of a down regulation signal pathway, or modulating this receptor from the cell surface. It could also induce a subpopulation of T cells capable of suppressing other alloreactive or autoreactive subpopulations. Anti-CD4 antibodies may also act by inducing complement or antibody-dependent T cell lysis or by removal of the T cells from the blood stream or site of inflammation. Therefore the Fc-recptor binding characteristics of each antibody may be important to their function. Alternative strategies include the use of anti-CD4 antibodies that have been radiolabeled or coupled to toxins.

These immunosuppressive properties of these anti-CD4 antibodies provide a therapeutic use in the suppression of activated T lymphocytes that mediate the diseases associated with transplanation and autoimmunity. The CD4 molecule is also the receptor for the gp120 subunit of the HIV virus. Since OKT4A inhibits the binding of gp120 to CD4, this antibody or fragments thereof may block viral infection.

The CD4 molecule is normally involved in providing a co-stimulatory signal to the T cell as a result of its binding to the HLA class II molecule. Therefore it is also possible that anti-CD4 antibodies can provide a co-stimulatory function in combination with other signal inducing reagents. This therapeutic strategy may be useful in the treatment of immunocompromised patients.

The CDR-grafted antibodies of the present invention may also be used in combination with other antibodies, particularly MAbs reactive with other markers on human cells responsible for the diseases. For example, suitable T-cell markers can include those grouped into the so-called "Clusters of Differentiation," as named by the First International Leukocyte Differentiation Workshop [36].

Generally, the present CDR-grafted antibodies will be utilized in purified form together with pharmacologically appropriate carriers. Typically, these carriers include aqueous or alcoholic/aqueous solutions, emulsions or suspensions, including saline and buffered media. Parenteral vehicles include sodium chloride solution, Ringer's dextrose, dextrose and sodium chloride and lactated Ringer's. Suitable physiologically acceptable adjuvants, if necessary to keep the complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, can also be present [37].

The CDR-grafted antibodies of the present invention may be used as separately administered compositions or in conjunction with other agents. These can include various immunotherapeutic drugs, such as cyclosporine, methotrexate, adriamycin or cisplatinum, and immunotoxins. Pharmaceutical compositions can include "cocktails" of various cytotoxic or other agents in conjunction with the CDR-grafted antibodies of the present invention, or even combinations of CDR-grafted antibodies according to the present invention and CDR-grafted antibodies having different specificities.

The route of administration of pharmaceutical compositions according to the invention may be any of those commonly known to those of ordinary skill in the art. For therapy, including without limitation immunotherapy, the CDR-grafted antibodies of the invention can be administered to any patient in accordance with standard techniques. The administration can be by any appropriate mode, including parenterally, intravenously, intramuscularly, intraperitoneally, or also, appropriately, by direct infusion with a catheter. The dosage and frequency of administration will depend on the age, sex and condition of the patient, concurrent administration of other drugs, counter indications and other parameters to be taken into account by the clinician.

The CDR-grafted antibodies of this invention can be lyophilized for storage and reconstituted in a suitable carrier prior to use. This technique has been shown to be effective with conventional immunoglobulins and art-known lyophilization and reconstitution techniques can be employed. It will be appreciated by those skilled in the art that lyophilization and reconstitution can lead to varying degrees of antibody activity loss (e.g. with conventional immunoglobulins, IgM antibodies tend to have greater activity loss than IgG antibodies) and that use levels may have to be adjusted to compensate.

The compositions containing the present CDR-grafted antibodies or a cocktail thereof can be administered for prophylactic and/or therapeutic treatments. In certain therapeutic applications, an adequate amount to accomplish at least partial inhibition or killing of a population of selected cells is defined as a "therapeutically-effective dose". Amounts needed to achieve this dosage will depend upon the severity of the disease and the general state of the patient's own immune system, but generally range from 0.005 to 5.0 mg of CDR-grafted antibody per kilogram of body weight, with doses of 0.05 to 2.0 mg/kg/dose being more commonly used. For prophylactic applications, compositions containing the present CDR-grafted antibody or cocktails thereof may also be administered in similar or slightly lower dosages.

A composition containing a CDR-grafted antibody according to the present invention may be utilized in prophylactic and therapeutic settings to aid in the alteration, inactivation, killing or removal of a select T cell target population in a mammal.

In another embodiment, the constructs described herein may be used extracorporeally or *in vitro* selectively to kill, deplete or otherwise effectively remove the target cell population from a heterogenous collection of cells. Blood from the mammal may be combined extracorporeally with the CDR-grafted antibodies whereby the undesired cells are killed or otherwise removed from the blood for return to the mammal in accordance with standard techniques.

In addition to the therapeutic uses, the CDR-grafted antibodies will find use in diagnostic assays. The CDR-grafted antibodies may be labelled in accordance with techniques known to the art. The CDR-grafted antibodies are also suitable for other *in vivo* purposes. For example, the CDR-grafted antibodies can be used for selective cell treatment of peripheral blood cells where it is desired to eliminate only target T lymphocytes or similarly in cell culture to eliminate unwanted T lymphocytes.

The present invention is now described, by way of example only, with reference to the accompanying drawings, in which:
Figure 1 depicts the nucleotide sequence of the OKT4A heavy chain variable domain;
Figure 2 depicts the nucleotide sequence of the OKT4A light chain variable domain;
Figure 3 depicts the OKT4A heavy chain variable domain amino acid sequence in which the CDRs are underlined;
Figure 4 depicts the OKT4A light chain variable domain amino acid sequence in which the CDRs are underlined;
Figure 5 depicts the alignment of KOL with the OKT4A CDR-grafted heavy chain amino acid sequence in which the CDRs are underlined, human sequences are in upper case and murine sequences are in lower case;
Figure 6 depicts the alignment of REI with the OKT4A CDR-grafted light chain amino acid sequence in which the CDRs are underlined, human sequences are in upper case and murine sequences are in lower case;
Figure 7 depicts the DNA sequence and amino acid translation of a CDR-grafted heavy chain;
Figure 8 depicts the DNA sequence and amino acid translation of a CDR-grafted light chain;
Figure 9 depicts the construction of a CDR-grafted OKT4A heavy chain expression vector;
Figure 10 depicts binding and blocking assays of CDR-grafted OKT4A light chain constructs in combination with a chimeric OKT4A heavy chain;
Figure 11 depicts binding and blocking assays of the OKT4A heavy chain constructs, HCDR1, HCDR2 and HCDR3, in combination with OKT4A light chain;
Figure 12 depicts the alignment of REI with the CDR-grafted OKT4A light chains, LCDR1 and LCDR2, and the murine OKT4A light chain amino acid sequences in which the CDRs are underlined, human sequences are in upper case and murine sequences are in lower case;
Figure 13 depicts the alignment of KOL with the CDR-grafted OKT4A heavy chains, HCDR1 through HCDR10, and the murine OKT4A heavy chain amino acid sequences in which the CDRs are underlined, human sequences are in upper case and murine sequences are in lower case;
Figure 14 depicts binding and blocking assays of the CDR-grafted heavy chain constructs, HCDR1, HCDR2 and HCDR3 in combination with the CDR-grafted light chain LCDR2; Figure 15 depicts binding and blocking assays of the CDR-grafted heavy chain constructs HCDR4 through HCDR10 in combination with the light chain LCDR2;
Figure 16 (A&B) depicts blocking assays of the OKT4A heavy chain constructs HCDR5, HCDR6, and HCDR10 in combination with light chain constructs LCDR2, LCDR3, LCDR2Q, LCDR3Q, and LCDR4Q, and the chimeric form of OKT4A;
Figure 17 depicts relative affinity assays of the OKT4A heavy chain constructs HCDR5 and HCDR10 in combination with light chain construct LCDR2 and the chimeric and murine forms of OKT4A using the murine and chimeric forms of OKT3 as negative controls;
Figure 18 depicts the results of studies on inhibition of MLR by various antibodies using T6 as negative control; and
Figure 19 depicts the results of studies on inhibition of proliferation by various antibodies.

### Humanization of OKT4A

OKT4A is a murine monoclonal antibody which recognizes the CD4 antigen located primarily on helper T lymphocytes. CDR-grafted antibodies have been constructed in which the CDRs of the variable domains of both heavy and light chains were derived from the murine OKT4A sequence. The variable domain frameworks and constant domains were derived from human antibody sequences.

The three CDRs that lie on both heavy and light chains are composed of those residues which structural studies have shown to be involved in antigen binding. Theoretically, if the CDRs of the murine OKT4A antibody were grafted onto human frameworks to form a CDR-grafted variable domain, and this variable domain were attached to human constant domains, the resulting CDR-grafted antibody would essentially be a human antibody with the specificity of murine OKT4A to bind the human CD4 antigen. Given the highly "human" nature of this antibody, it would be expected to be far less immunogenic than murine OKT4 when administered to patients.

Following testing for antigen binding of a CDR-grafted OKT4A antibody in which only the CDRs were grafted onto the human framework, it was shown that this did not produce a CDR-grafted antibody having reasonable affinity for the CD4 antigen. It was therefore decided that additional residues adjacent to some of the CDRs and critical framework residues needed to be switched from the human to the corresponding murine OKT4A residues in order to generate a functional antibody.

### Isolation of the OKT4A heavy and light chain cDNA and DNA sequence analysis of the variable domain.

To design the CDR-grafted OKT4A antibody, it was first necessary to determine the sequence of the variable domain of the murine OKT4A heavy and light chains. The sequence was determined from heavy and light chain cDNA that had been synthesized from the respective mRNA.

mRNA was prepared from OKT4A-producing hybridoma cells by guanidinium thiocyanate extraction followed by cesium chloride gradient purification [38].- cDNA was synthesized and libraries were prepared and screened in Dr. J. Rosen's laboratory at The R.W. Johnson Pharmaceutical Research Institute in La Jolla, California. cDNA was synthesized from the mRNA, EcoRI linkers were added, and it was then ligated into the EcoRI site of the lgt10 cloning vector. The recombinant phage was packaged into infectious particles which were used to infect *E*. *Coli* C600.

This library was screened for OKT4A heavy chain sequences using oligonucleotide probes Cg and FR3. Cg (the mRNA sequencing primer from Pharmacia LKB Biotechnologies, Inc) has the sequence 5' GGCCAGTGGATAGAC 3' and binds to the murine IgG constant domain. Probe FR3 has the sequence 5' GGCCGTGTCCTCAGACCT 3' and binds to the third framework region of the variable domain of murine heavy chains. Five positive clones were evaluated by southern transfer and hybridization to probes Cg, FR3 and a cDNA to mouse IgG2a CH3. A single clone with a 1600 bp EcoRI insert which hybridized to all three probes was selected.

The library was screened for the OKT4A light chain sequence using an oligonucleotide probe Ck (the mRNA sequencing primer from Pharmacia) with a sequence of 5' GGCTCCAGGTTGCTGATGCTGAAGG 3' and which binds to the mouse kappa constant domain. Six positive clones were further assessed by southern transfer and hybridization to oligonucleotide probes T4AK, whose sequence is 5' GGCTCCAGGTTGCTGATGCTGAAGG 3' and which binds to mouse kappa chain framework region 3, and Ck. A single clone which contained a 900 bp EcoRI insert and hybridized to both probes was chosen.

The 1600 bp heavy chain cDNA was subcloned into the EcoRI sites of the pBluescript plasmid vector (Stratagene Cloning Systems) and the M13mp8 sequencing vector (Pharmacia LKB Biotechnologies, Inc). The 900 bp light chain cDNA was subcloned into the EcoRI sites of the plasmid vector pUC8 (Pharmacia LKB Biotechnologies, Inc) and the M13mp19 sequencing vector.

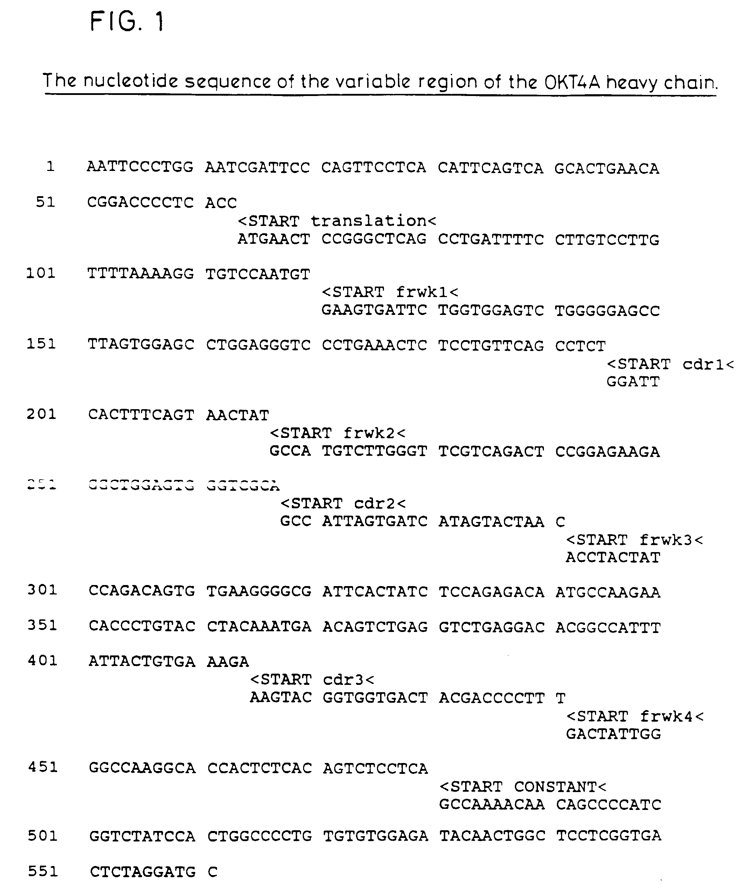

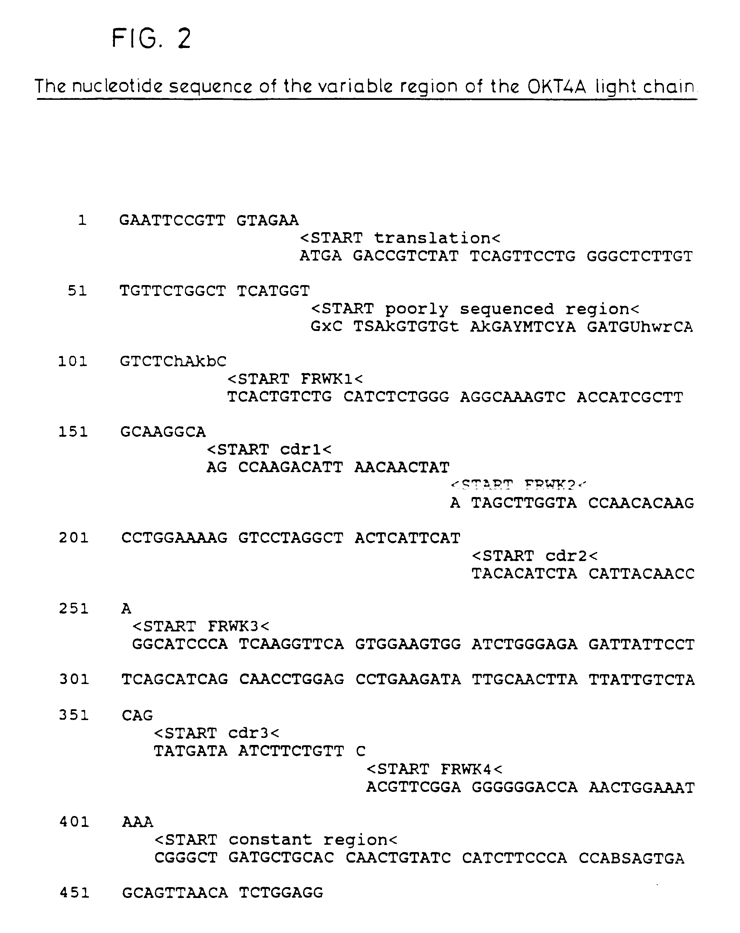

The dideoxy-nucleotide chain termination method of DNA sequence analysis [39] was used to determine sequence of both single-stranded (M13) and double-stranded (plasmid) templates. The sequence of the 5' untranslated regions, signal sequences, variable domains and a portion of the constant domains were determined for both heavy and light chain cDNA. The DNA sequence for heavy and light chains is illustrated in Figures 1 and 2. The amino acid translation of the heavy chain variable domain sequence is presented in Figure 3. A translation of the light chain variable domain is presented in Figure 4.

It is to be noted that the nucleotide sequence given for the light chain has an A residue at position 163, towards the beginning of the CDR1 coding sequence (see Figure 2).

Translation of this sequence gives a glutamine residue at position 27 in the light chain (see Figure 4).

When the sequencing of the OKT4 light chain was originally carried out, nucleotide residue 163 was thought to be a C residue, giving a proline residue at position 27 in the light chain. The first CDR-grafted antibodies produced by the present inventors were constructed on the assumption that light chain residue 27 was a proline residue. This can be seen from Figures 6, 8 and 12.

### Design of the CDR-grafted OKT4A Antibody

To design the CDR-grafted OKT4A antibody, it was necessary to determine which residues of murine OKT4A comprise the CDRs of the light and heavy chains. Examination of antibody X-ray crystal structures shows the antigen binding surface to be located on a series of three loops extending from the b-barrel framework of the variable domain. These loops can thus be used to define the CDRs. Since the crystal structure of murine OKT4A is not available, the structure of a similar murine antibody of known crystal structure was used to define the residues of the loops.

Three regions of hypervariability amid the less variable framework sequences are found on both light and heavy chains [2]. In most cases these hypervariable regions correspond to, but may extend beyond, the CDRs. It was decided that a combination of those murine OKT4A residues in the CDRs and those in the hypervariable regions would comprise composite CDRs to be grafted onto the human antibody framework. The amino acid sequences of the murine OKT4A heavy and light chains are presented in Figures 3 and 4, with the selected composite CDRs underlined.

The human antibody framework sequence for the heavy chain is that of the human antibody KOL. KOL was chosen because its X-ray crystallographic structure had been determined to a high degree of resolution. This should allow for accurate molecular modelling of the antibody. For the same reason, the framework sequence of the human light chain dimer, REI, was used for the light chain frameworks. The amino acid sequences of KOL and REI are shown in Figures 5 and 6 in comparison to those of the CDR-grafted OKT4A heavy (HCDR1) and light (LCDR1) chain variable domains.

The CDR-grafted heavy chain was designed to have a human IgG4 constant portion. The IgG4 subclass was selected based on experience with the murine anti-CD3 monoclonal antibody, OKT3, which is used to treat renal graft rejection. OKT3 has a murine IgG2a isotype and does not fix complement in humans. The human IgG4 isotype also does not fix complement. The CDR-grafted OKT4A light chain was constructed with the human kappa constant domain.

### Construction of the CDR-grafted OKT4A genes

Heavy and light chain CDR-grafted variable domains were constructed by the ligation of synthetic double-stranded DNA oligomers, similar to the method employed in [40]. The 5' end of the variable domains contain signal sequences of the light and heavy chains of the murine monoclonal antibody B72.3 [41]. The signal sequence directs secretion of the antibody from mammalian cells. A Kozak sequence [42] immediately precedes the AUG start codon to enhance translation. The variable domains were then ligated to DNA coding for the human constant domains to create the CDR-grafted heavy and light chain genes.

### CDR-Grafted OKT4A Heavy Chain Construction

Eight complementary pairs of oligomers, approximately 30 bp in length were designed to have overlapping ends and to span the variable domain from a XhoI site located in framework 2 to a HindIII site at the beginning of the first constant domain. These eight oligomer pairs were synthesized, ligated together in a step-wise manner and then ligated to the HindIII 5' end of the human IgG4 constant domain DNA. The IgG4 DNA was provided by Celltech, Ltd (Slough, U. K.) as genomic DNA. It is a 2153bp insert in an M13 phage DNA vector with a 5' EcoRI and a 3' BamHI restriction site. The CH1, hinge, CH2 and CH3 domains are surrounded by four introns. The gene was modified by Celltech to have a C to A change at the penultimate base of the CH1 exon to create a new HindIII site for CDR-grafted gene construction purposes.

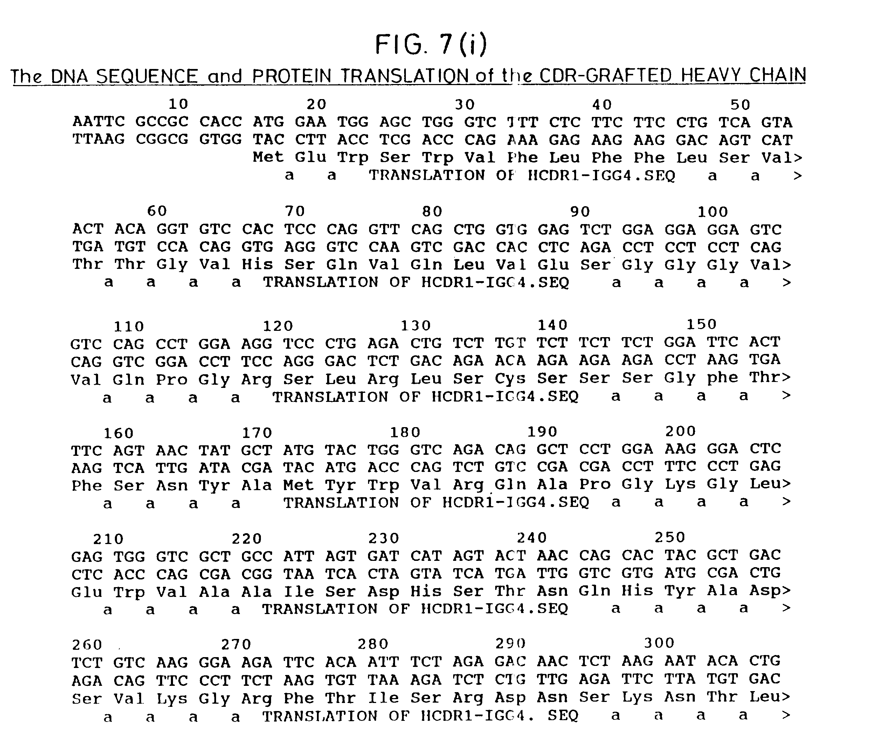

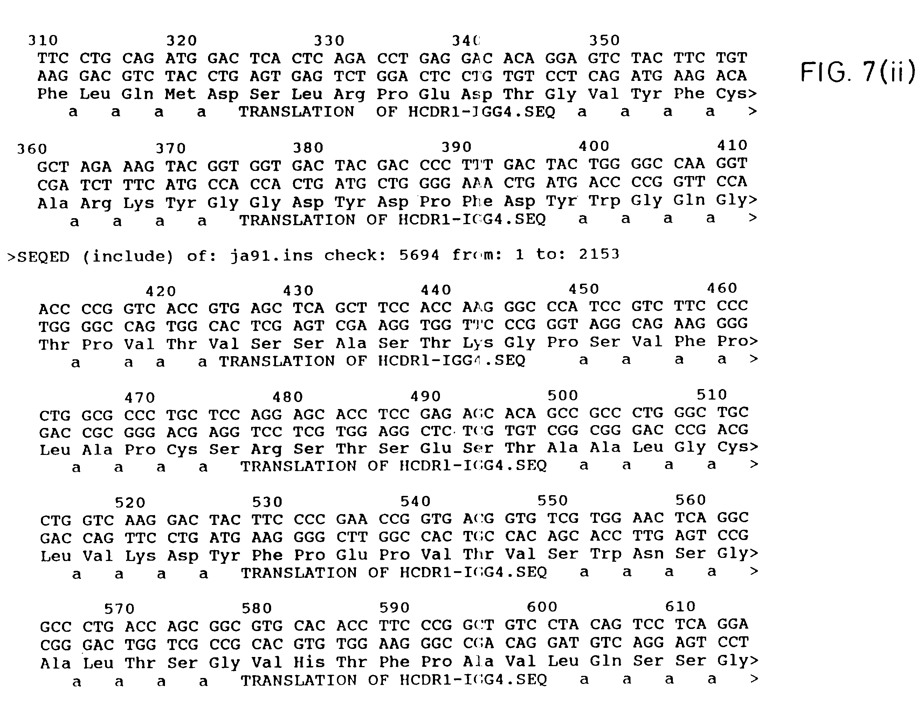

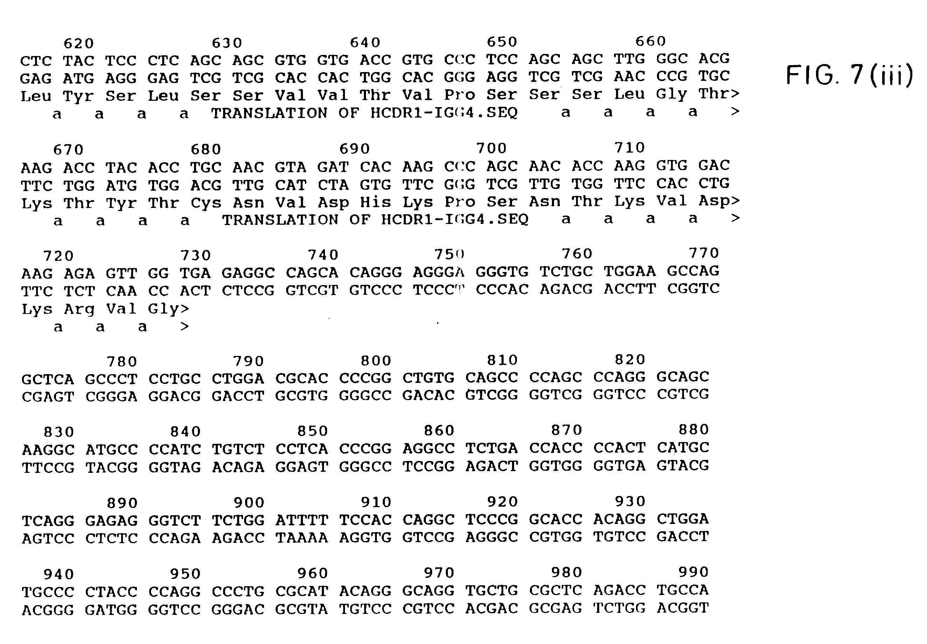

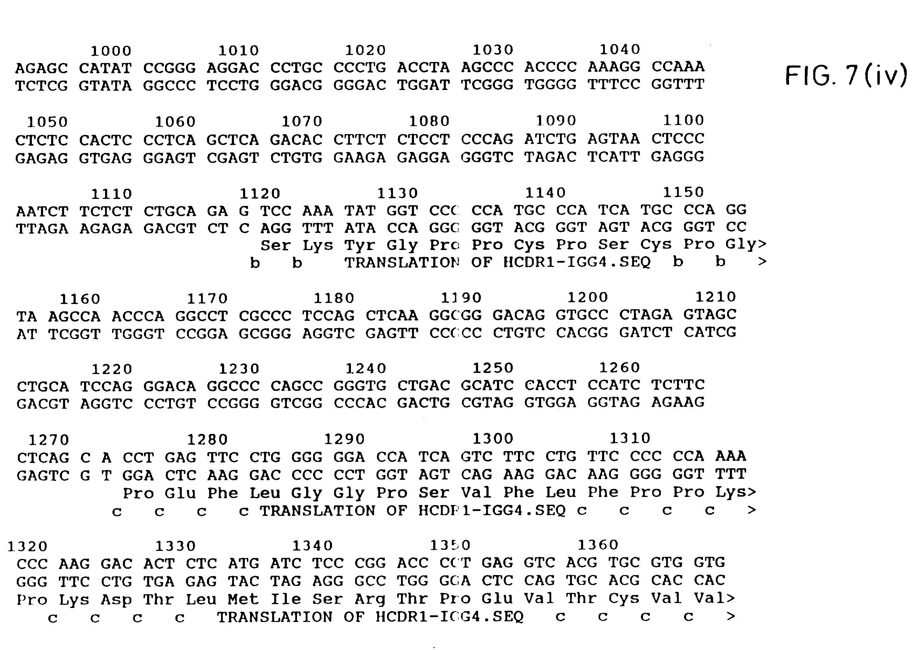

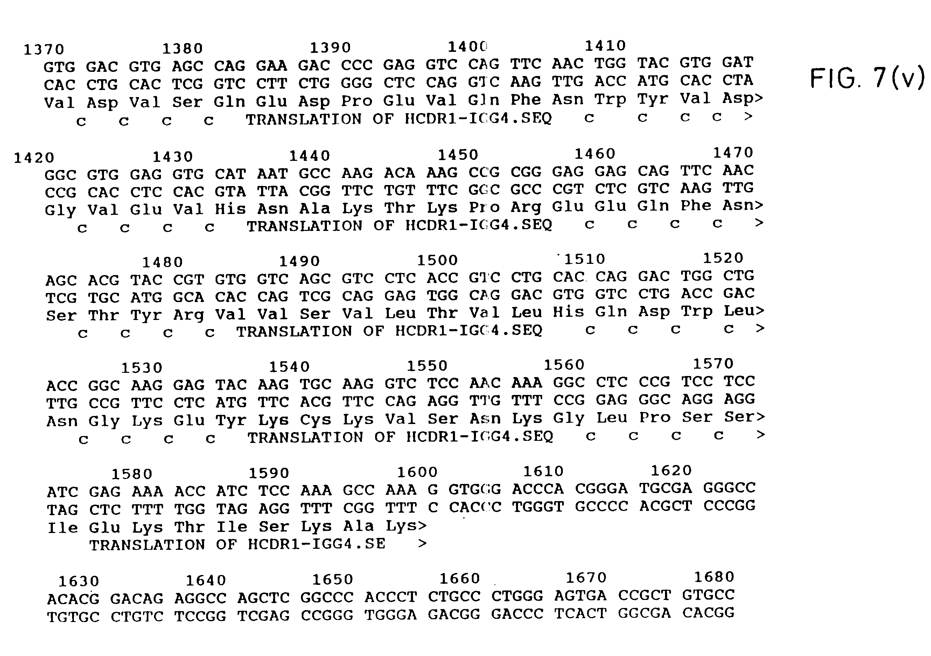

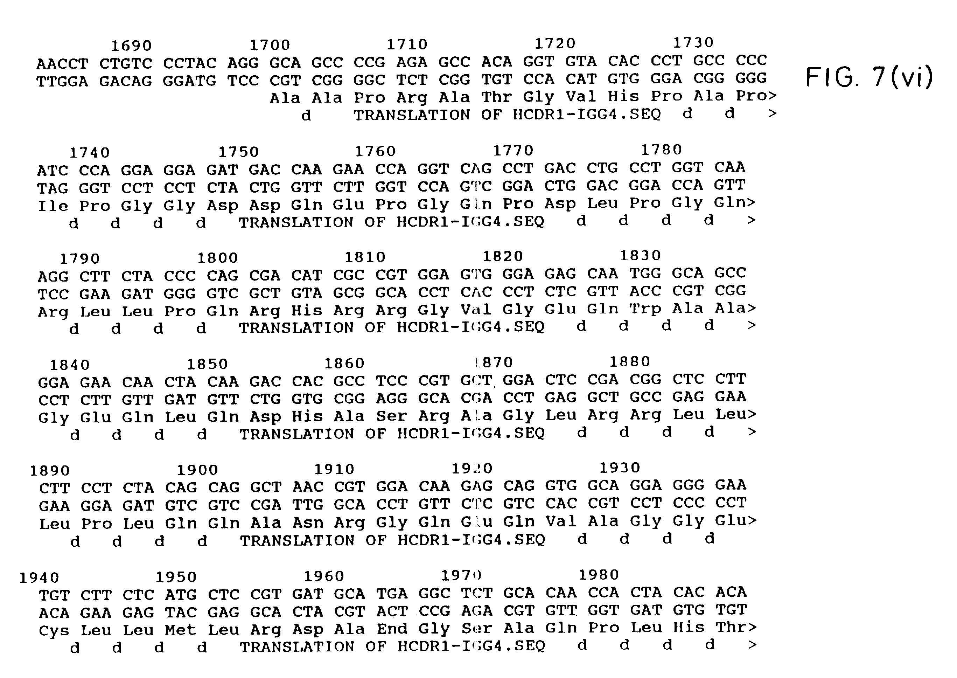

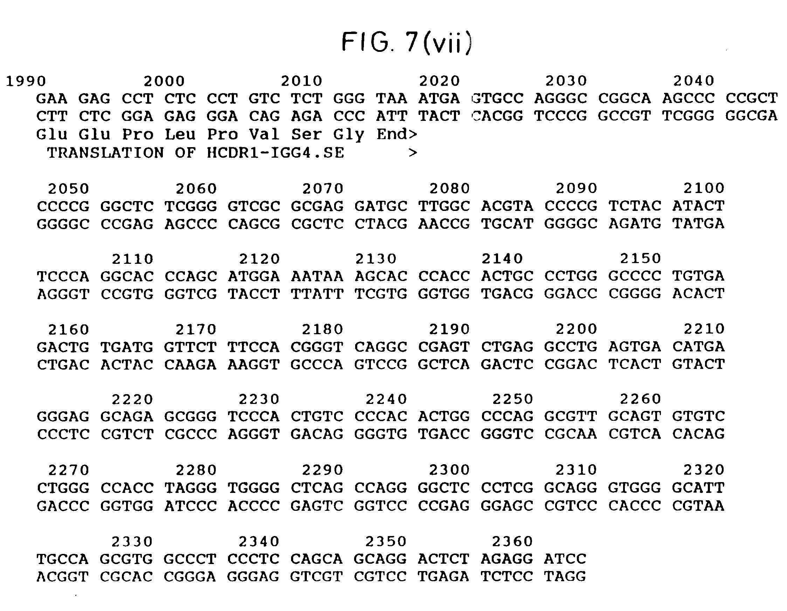

The 5' end of the variable domain was constructed by ligating two complementary pairs of synthetic oligomers, each approximately 90bp in length. This fragment, which had a 5' EcoRI end and a 3' XhoI end was ligated to the XhoI end of the fragment described above to yield the complete CDR-grafted heavy chain gene. This gene is 2364bp in length and has a 5' EcoRI end and a 3' BamHI end. The DNA sequence with amino acid translation of the gene is shown in Figure 7. The regions of interest, defined by nucleotide number are:

| | |
|---|---|
| 1 - 14 | EcoRI site and Kozak sequence |
| 15 - 71 | signal sequence |
| 72 - 146 | framework 1 |
| 147 - 176 | CDR1 |
| 177 - 218 | framework 2 |
| 219 - 254 | CDR2 |
| 255 - 362 | framework 3 |
| 363 - 392 | CDR3 |
| 393 - 431 | framework 4 |
| 432 - 727 | CH1 domain |
| 728 - 1117 | intron |
| 118 - 1153 | hinge domain |
| 1154 - 1271 | intron |
| 1272 - 1599 | CH2 domain |
| 1600 - 1698 | intron |
| 1699 - 2016 | CH3 domain |
| 2017 - 2366 | 3' untranslated region |

### CDR-grafted light chain gene construction

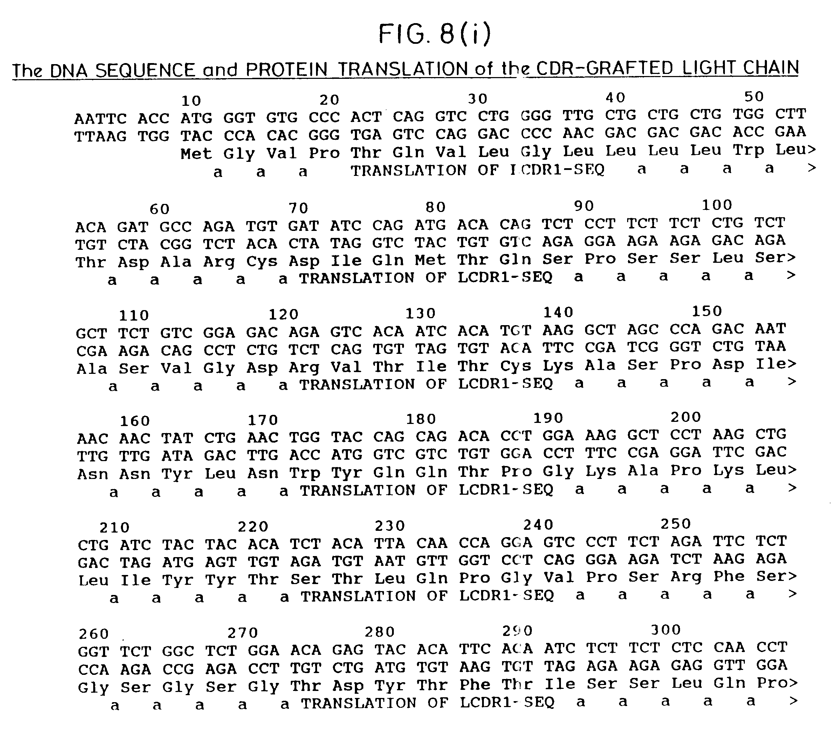

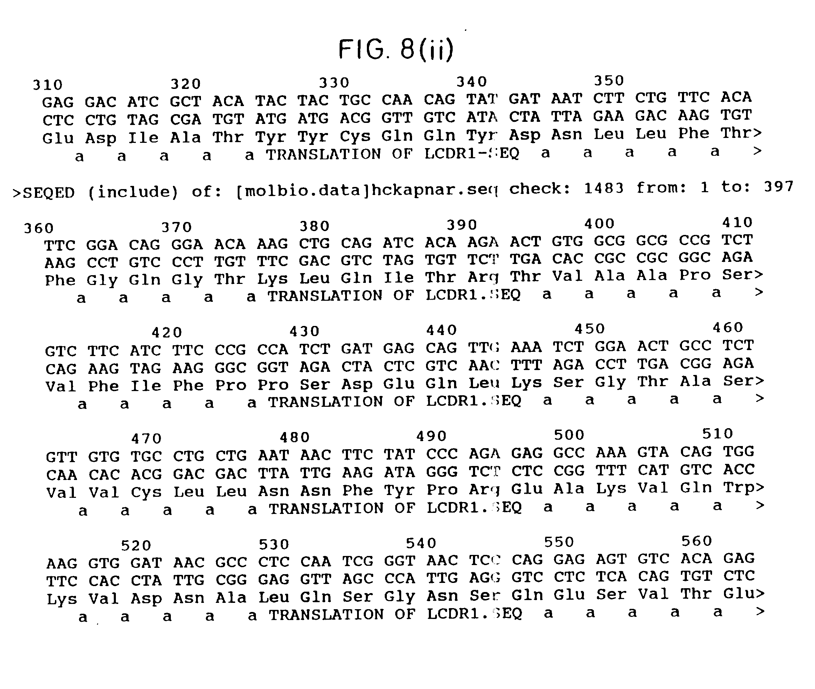

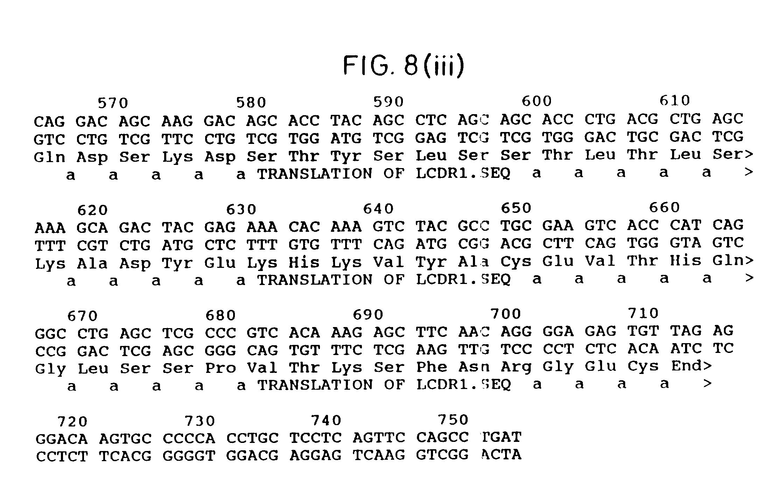

Twelve complementary pairs of synthetic oligomers with overlapping ends were ligated simultaneously to assemble the CDR-grafted light chain variable domain. This fragment had a 5' EcoRI end and a 3' NarI end. This was ligated to the 5' NarI end of the human kappa constant domain DNA. Human kappa constant cDNA was modified by Celltech to include a NarI restriction site in the third and fourth codons. The resulting CDR-grafted light chain gene was 754bp in length and had EcoRI ends. The DNA sequence with amino acid translation is shown in Figure 8. The regions of interest defined by nucleotide number are:

| | |
|---|---|
| 1 - 8 | EcoRI site and Kozak sequence |
| 9 - 68 | signal sequence |
| 69 - 143 | framework 1 |
| 144 - 164 | CDR1 |
| 165 - 215 | framework 2 |
| 216 - 236 | CDR2 |
| 237 - 338 | framework 3 |
| 339 - 356 | CDR3 |
| 357 - 404 | framework 4 |
| 405 - 710 | kappa constant domain |
| 711 - 754 | untranslated |

### Expression of the CDR-grafted OKT4A antibody

### Construction of the Heavy Chain Expression Vector

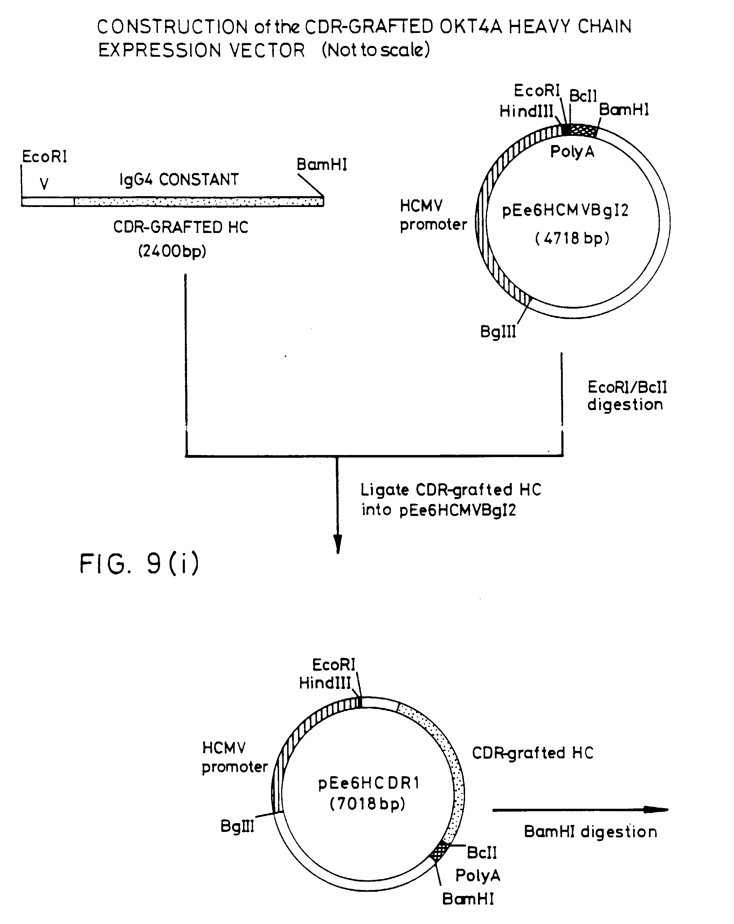

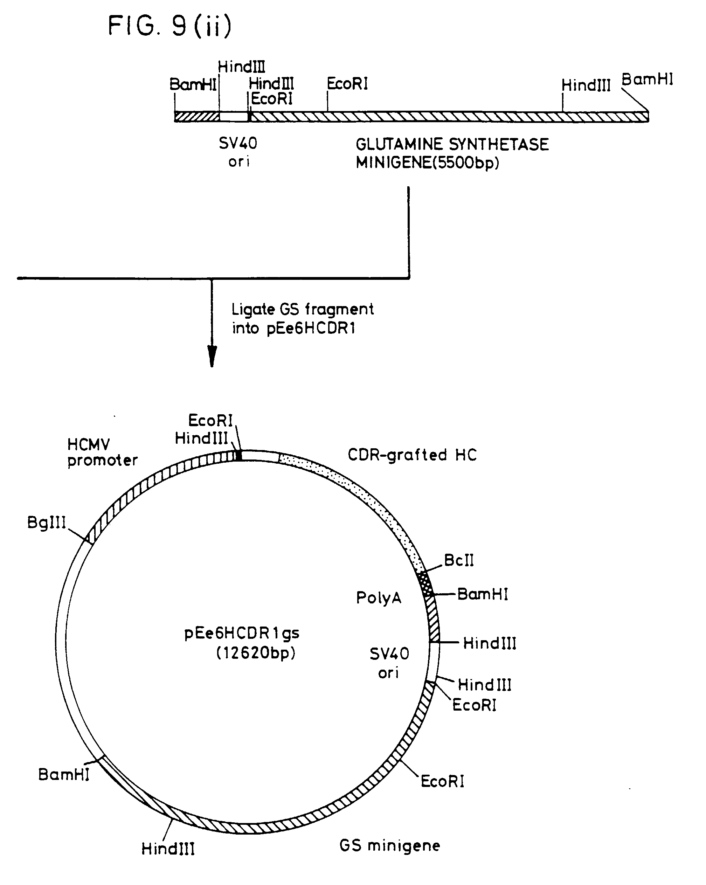

A CDR-grafted heavy chain expression vector was constructed by inserting the heavy chain gene into the expression plasmid pEe6HCMVBgl2 and the adding the GS fragment, which is composed of the SV40 origin and glutamine synthetase minigene. These steps are diagrammed in Figure 9. pEe6HCMVBgl2 and the GS fragment were provided by Celltech, Ltd.

pEe6HCMV was digested at the EcoRI and BclI sites. The pEe6HCMVBgl2 DNA had been demethylated by passaging it through the DAM *E. Coli* strain GM242, which lacks the deoxyadenosine methylase. BclI will only restrict DNA which does not contain N⁶-methylated deoxyadenosine at the enzyme's recognition site. The overhang resulting from the BclI restriction is compatible with the BamHI overhang. The EcoRI/BamHI CDR-grafted heavy chain gene (HCDR1) was then ligated to the EcoRI/BclI ends of pEe6HCMVBgl2 to produce pEe6HCDR1.

A 5500 bp BamHI fragment containing the glutamine synthetase minigene and the SV40 origin of replication and early and late promoters was inserted into the BamHI site of pEe6HCDR1 to produce pEe6HCDRlgs. The correct orientation of the GS fragment was verified by restriction analysis. pEe6HCDRlgs was prepared for mammalian cell transfection by the alkaline lysis method and cesium chloride gradient purification [43].

pEe6HCDRlgs is capable of expressing the CDR-grafted OKT4A heavy chain in COS and CHO cells. The HCMV promoter lies 5' to the heavy chain gene and directs its transcription. The SV40 polyadenylation signal sequence, which lies 3' to the gene, acts as a transcriptional terminator. For transient expression in COS cells, the SV40 origin of replication is present in the GS 5500 bp fragment. The GS minigene is present as a selectable marker for use following CHO cell transfections. Expression of the glutamine synthetase minigene is driven by the SV40 late promoter. The GS fragment is oriented such that the SV40 late promoter drives transcription in the same direction as the HCMV promoter.

Several post-transcriptional events occur to produce the CDR-grafted heavy chain. Within the nucleus, the three intervening sequences of the IgG4 constant portion are removed and the exons are spliced together to create a mature mRNA. Following translation, the 19 amino acid signal sequence is removed in the rough endoplasmic reticulum (ER). A single carbohydrate is added to the CH2 domain of each chain in the ER and the Golgi apparatus. Each chain also contains four intrachain disulfide bonds. When a light chain peptide is provided by a cotransfected light chain expression vector, a mature antibody is assembled by the binding together, via disulfide bonds, of two heavy and two light chains.

### Construction of the CDR-grafted OKT4A light chain expression vector:

The CDR-grafted OKT4A light chain expression vector was constructed by inserting the CDR-grafted light chain gene into the expression vector pEe6HCMVBgl2 and then adding the SV40 origin and glutamine synthetase minigene-containing GS fragment. The light chain expression vector was constructed by essentially the same process that was used for the heavy chain expression vector as illustrated in Figure 9. The light chain gene was ligated into the EcoRI site of pEe6HCMVBgl2 to produce pEe6LCDR1. The correct orientation of the light chain gene was verified by restriction analysis. The 5500 bp GS fragment was inserted into the BamHI site to produce pEe6LCDRlgs. The correct orientation of the GS fragment was verified by restriction analysis. pEe6LCDRlgs prepared for mammalian cell transfection by the alkaline lysis method [43] and by cesium chloride gradient purification.

As with the CDR-grafted heavy chain gene, the transcription of the CDR-grafted light chain gene in pEe6LCDRgs is driven by the HCMV promoter and transcriptional termination is signalled by the SV40 polyadenylation signal sequence. The SV40 origin of replication contained in the GS fragment allows for autonomous replication of this construct in COS cells. The glutamine synthetase minigene in the GS fragment provides a mechanism for selection and amplification in CHO cells.

Post-transcriptional processing of the CDR-grafted light chain mRNA is not required prior to translation because no introns are present in the gene. Following translation, the leader sequence is removed in the rough ER. Two intrachain disulfide bonds are formed. Assembly of a mature antibody was discussed in the previous section.

### Transient expression of CDR-grafted OKT4A in COS-1 cells

The transient expression of the CDR-grafted genes in COS-1 cells provides a rapid and convenient system to test CDR-grafted OKT4A antibody expression and function. COS-1 cells constituitively express the SV40 large T antigen which supports the transient replication of episomes carrying the SV40 origin of replication [44]. The CDR-grafted gene expression vectors pEe6HCDRlgs and pEe6LCDRlgs contain the SV40 origin of replication as a portion of the GS fragment. Upon transfection into COS-1 cells, the expression vectors are replicated in the nucleus to a high copy number, resulting in relatively high expression levels.

COS-1 cells were obtained from the American Type Culture Collection (CRL 1650) and cultured in Dulbecco's Modified Eagle Medium (DMEM from GIBCO) with 10% fetal calf serum. The CDR-grafted gene expression vectors were transfected into COS cells using the DEAE-dextran method followed by DMSO shock [45]. Briefly, 0.2 ml of 1 mg/ml DEAE-dextran in buffer is added to 15 mg vector DNA in 0.8 ml DMEM/Tris. This was added to 1 - 1.5 x 10⁶ cells in a 60 mm tissue culture plate and incubated for approximately 6 hours. The DEAE-dextran/DNA complex is removed and 10% DMSO in buffer is added to the plate for 2 minutes. This is removed, the cells are washed once with DMEM and then incubated with DMEM containing 10% fetal calf serum for 3-4 days. At that time supernatant from the wells is harvested and examined for antibody levels and ability to bind CD4 positive lymphocytes.

Antibody levels were determined by ELISA. Wells were coated with a goat anti-human Fc specific antibody. Various dilutions of the COS cell supernatant containing secreted antibody were added, incubated for one hour at room temperature in a humidity chamber and washed. A horse radish peroxidase-linked goat anti-human kappa chain antibody was added, incubated for one hour at room temperature and washed. Substrate for the horse radish peroxidase was added for detection. The CDR-grafted OKT4A levels following co-transfection of pEe6HCDRlgs and pEe6LCDRlgs range from 200 to 1200 ng/ml of COS cell supernatant.

### Antigen Binding Studies

CDR-grafted OKT4A produced by COS cells was tested for its ability to bind to human peripheral blood lymphocytes (PBLs) or the CD4-positive HPBALL (human peripheral blood acute lymphocytic leukemia) cell line. It was also tested for its ability to block the binding of murine OKT4A to these cells. Binding was measured by the following procedure. PBLs were isolated from serum or HPBALL cells were harvested from tissue culture. Cells were incubated at 4°C for 1 hour with various dilutions of test antibody, positive control antibody or negative control antibody. The cells were washed once and incubated at 4°C for 1 hour with an FITC-labeled goat anti-human IgG (Fc-specific, mouse absorbed). The cells were washed twice and analyzed by cytofluorography. chimeric OKT4A (described below) was used as a positive control. FITC-labeled murine OKT4A was used as a positive control for direct binding. Cells incubated with mock-transfected COS cell supernatant, followed by the FITC-labeled goat anti-human IgG, provided the negative control.

To test the ability of CDR-grafted OKT4A to block murine OKT4A binding, the PBLs or HPBALL cells were incubated at 4°C for 1 hour with various dilutions of test antibody or control antibody. A fixed saturating amount of FITC-OKT4A was added. The samples were incubated for 1 hour at 4°C, washed twice and analyzed by cytofluorography. Positive controls were FITC-labeled OKT4A to determine maximum binding and unlabeled murine OKT4A as a reference standard for blocking. Negative controls were unstained cells with or without mock-transfected cell supernatant.

The ability of the CDR-grafted OKT4A light chain to bind CD4 positive cells and block the binding of murine OKT4A was initially tested in combination with a chimeric OKT4A heavy chain. The chimeric OKT4A heavy chain is composed of the murine OKT4AA variable domain and the human IgG4 constant portion. The chimeric heavy chain gene is expressed in the same expression vector used for the CDR-grafted genes. The CDR-grafted light chain expression vector and the chimeric heavy chain expression vector were co-transfected into COS cells. The full chimeric OKT4 antibody (chimeric light chain and chimeric heavy chain) was found to be fully capable of binding to CD4 positive cells and blocking the binding of murine OKT4 to these cells.

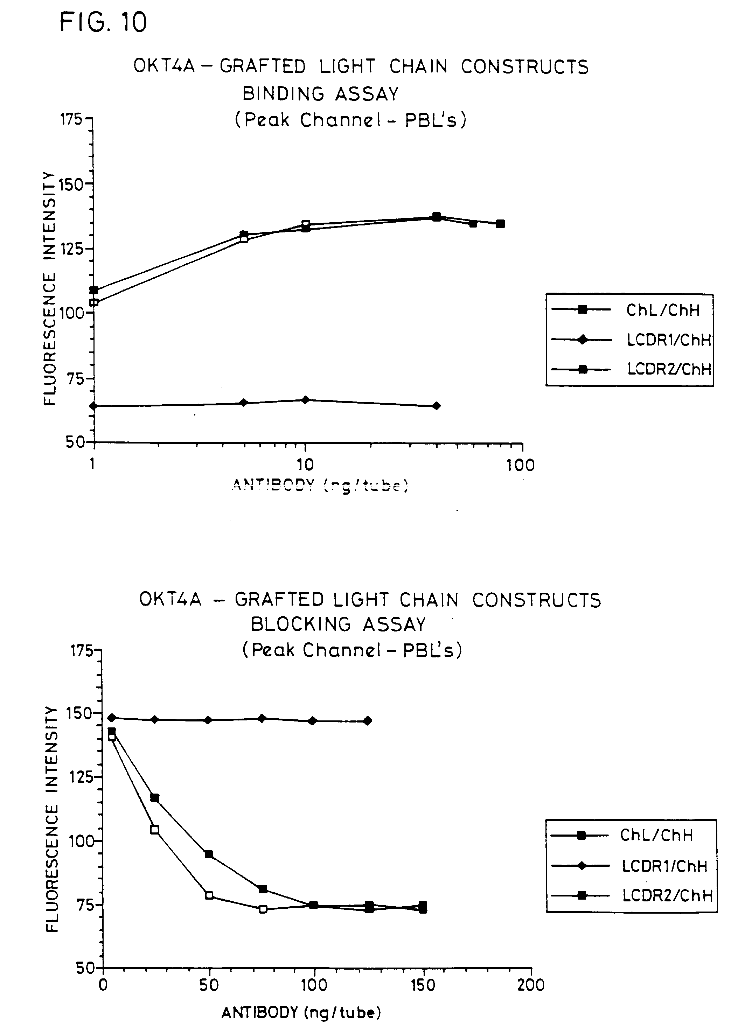

As Figure 10 illustrates, the CDR-grafted OKT4A light chain, LCDR1, in combination with the chimeric OKT4A heavy chain was unable to bind CD4 positive cells or block the binding of murine OKT4A to these cells.

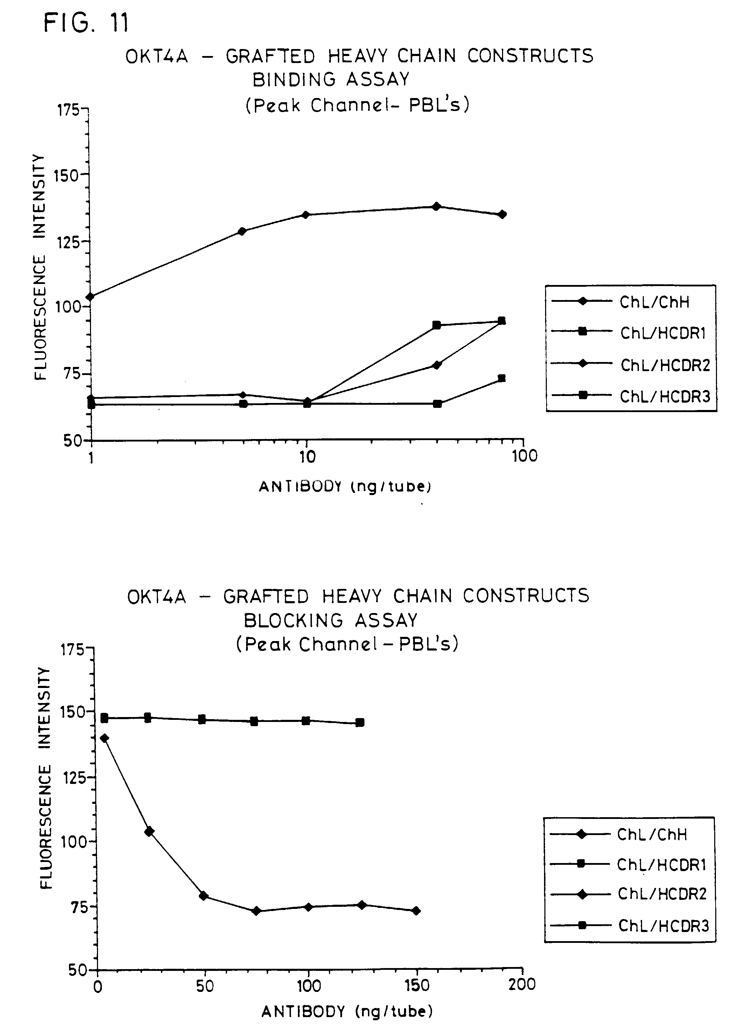

Figure 11 shows the binding and blocking studies done with the CDR-grafted OKT4A heavy chain, HCDR1, combined with the chimeric OKT4A light chain. The chimeric OKT4A light chain is composed of a murine OKT4A variable domain and a human kappa constant domain. It is also expressed in the same expression vector as is used for the CDR-grafted antibodies. COS cells were co-transfected with the CDR-grafted heavy chain expression vector and the chimeric light chain expression vector.

The CDR-grafted OKT4A heavy chain, HCDR1, in combination with the chimeric OKT4A light chain was also unable to bind CD4 positive cells or block the binding of murine OKT4A to these cells.

### Modification of the CDR-Grafted Antibody

The binding and blocking data clearly demonstrated that the initially designed CDR-grafted OKT4A antibody was not capable of recognizing the CD4 antigen. Further modification of the antibody was necessary. Either the murine OKT4A CDRs needed to be further expanded or critical framework residues involved in the positioning of CDRs, domain packing or light and heavy chain interactions needed to be changed from human to mouse.

Molecular modelling was used to identify the residues which appeared most critical for successful antigen interaction. Modelling was done at Celltech, Ltd with HYDRA software on a SiliconGraphics instrument.

### Modification of the CDR-Grafted Light Chain

The crystal structure of OKT4A has not been determined, so a molecular model of OKT4A itself could not be used in the analysis. To analyze residues of the CDR-grafted light chain, a molecular model of the human REI light chain was superimposed with a mouse MOPC 603 Fab fragment. The MOPC 603 light chain is similar in amino acid sequence to OKT4A. Also studies were done where the human REI light chain and the human KOL heavy chain were docked. Decisions were made to extend CDR1 by converting residues 33 and 34 from the human leu and asp to the murine OKT4A ile and ala. The human REI residue glu38 was found to be involved in heavy chain and light chain packing. Changing this to the murine OKT4A his38 may be beneficial. Residue 49 at the amino terminal edge of CDR2 directly impacts CDR2 and also makes contact with CDR3 of the heavy chain. Residue 89 near the amino terminal end of CDR3 interacts with phe98 in CDR3 of the light chain and also contacts CDR3 of the heavy chain. The REI tyr49 and gln89 were changed to the murine OKT4A his49 and leu89.

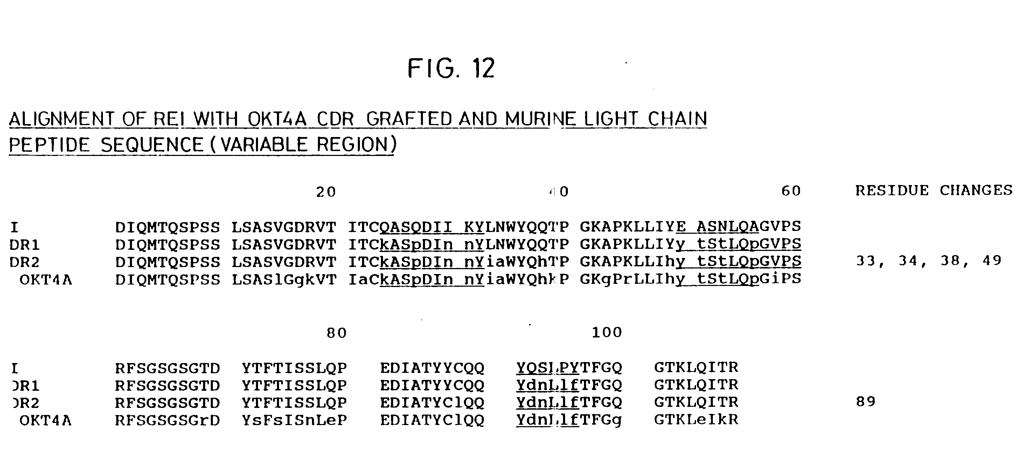

The new CDR-grafted OKT4A light chain gene that was generated by the above changes was designated LCDR2. A comparison of the amino acid sequence of the variable domains of the human REI, LCDR1, LCDR2, and the assumed murine OKT4A light chain is shown in Figure 12. The changes were effected by altering codons by site-directed mutagenesis [46]. The bluescript phagemid vector from Stratagene Cloning Systems was used to generate single-stranded template for mutagenesis. The expression vector pEe6LCDR2gs was constructed in the same manner as for LCDR1. COS cells were co-transfected with pEe6LCDR2gs and the chimeric heavy chain expression vector.

The results of binding and blocking studies are shown in Figure 10. The LCDR2 version of the CDR-grafted OKT4A light chain, in combination with the chimeric OKT4A heavy chain, is capable of binding to CD4 positive cells and of blocking the binding of murine OKT4A. These data show that LCDR2 is a functional CDR-grafted OKT4A light chain.

### Modification of the CDR-grafted heavy chain

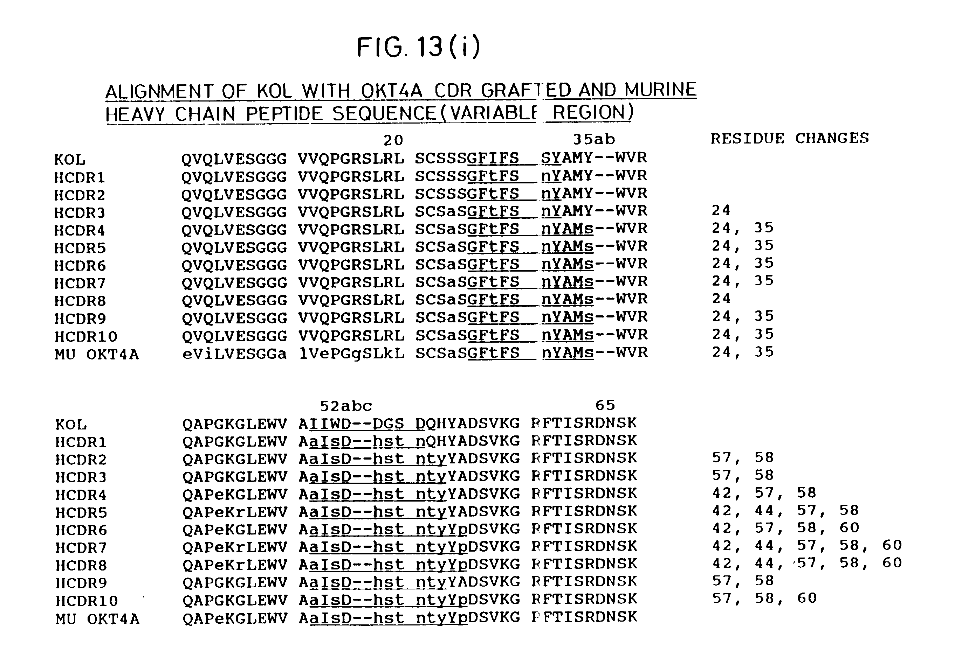

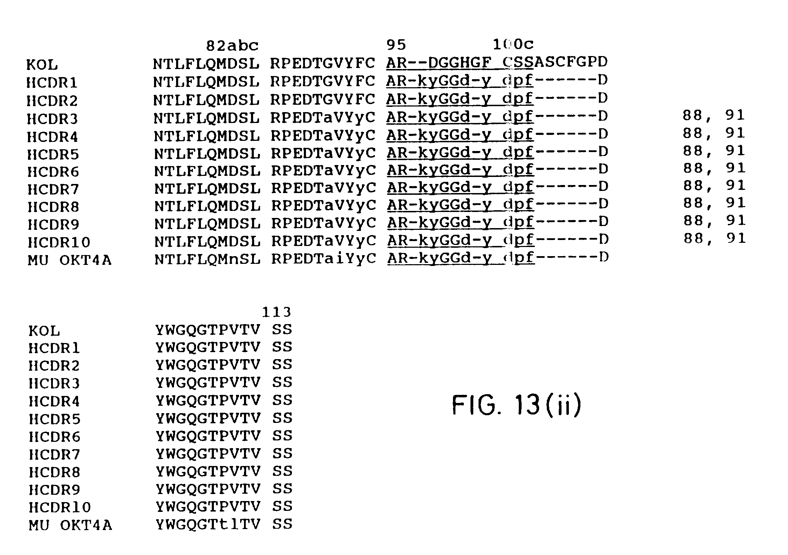

For modelling studies of the heavy chain the molecular model of the human antibody KOL was used. All residue changes were made by site-directed mutagenesis to change codons. A decision was made to change glu57 and his58 of KOL to thr57 and tyr58 of murine OKT4A. This revised CDR-grafted heavy chain was designated HCDR2. In addition to changes at residues 57 and 58, residue 24 lies near CDR1 and may be involved in positioning CDR1. Also residues 88 and 91 are involved in heavy chain variable domain packing and the interface between the heavy and light chains. These three additional residue changes from KOL to murine OKT4A were incorporated into the heavy chain version HCDR3. An amino acid sequence comparison of the variable domains of KOL, HCDR1, HCDR2, HCDR3, murine OKT4A heavy chain, and versions to be described below is illustrated in Figure 13.

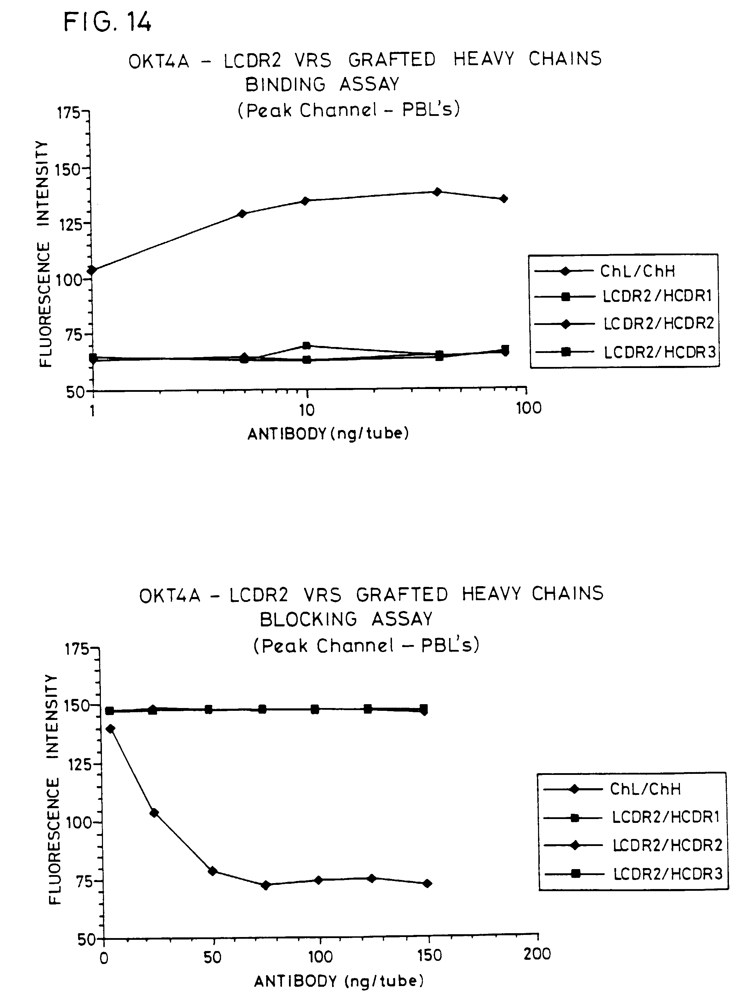

Expression vectors pEe6HCDR2gs and pEe6HCDR3gs were co-transfected into COS cells with either the chimeric OKT4A light chain expression vector or pEe6LCDR2gs. Binding and blocking data are presented in Figures 11 and 14. Neither HCDR2 nor HCDR3 was able to effectively interact with antigen when combined with a chimeric or CDR-grafted OKT4A light chain.

Further modifications to the CDR-grafted heavy chain were explored. A decision to change KOL tyr35 to murine OKT4A ser35 was made. Molecular modelling demonstrated that residue 42 was involved in positioning CDR2. Residue 44 is involved in light chain contacts. It may be beneficial to change the KOL gly42 and gly44 to murine OKT4A glu42 and arg44. KOL ala60 was changed to the murine OKT4A pro60. These changes were introduced in various combinations, while retaining the changes made at residues 24, 57, 58, 88, and 91 in the previous versions. These latter versions were denoted HCDR4, HCDR5, HCDR6, HCDR7, HCDR8. The residue changes in each are described in Figure 13. The same expression vector was used as with the other constructions. COS cells were co-transfected with the new heavy chain expression vectors and pEe6LCDR2gs.

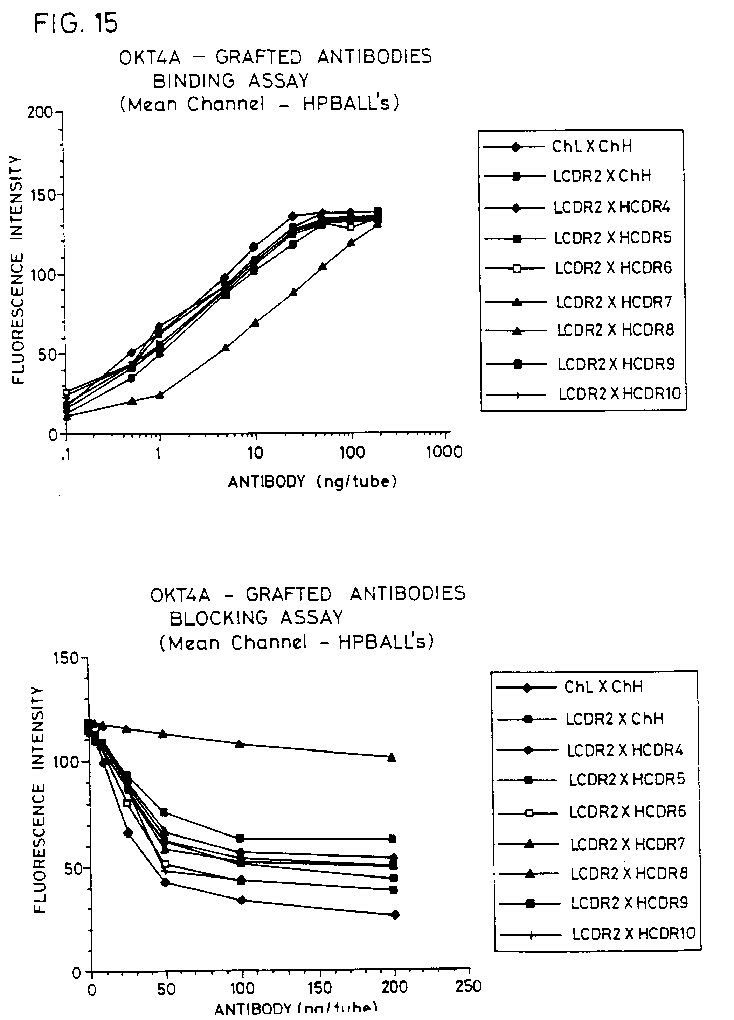

The results of binding and blocking studies, in combination with LCDR2 (Figure 15), show positive interactions with the CD4 antigen in all of these versions except HCDR8. Apparently the conversion of tyr35 to the murine ser35 is a critical change. The change to the 1.2 murine residue at position 60 appears to enhance antigen interaction (compare HCDR6 vs HCDR4) while the change at position 44 appears to be slightly inhibitory (HCDR5 vs HCDR4, and HCDR7 vs HCDR6).

To determine if changes at residues 35 and 60 would be sufficient for antigen binding, HCDR9 (murine residues at positions 24, 35, 57, 58, 88, 91) and HCDR10 (murine residues at positions 24, 35, 57, 58, 60, 88, 91) were generated by site-directed mutagenesis. The same expression vector system was used for these versions of the CDR-grafted heavy chain. They were co-transfected into COS cells with pEe6LCDR2gs.

The results of binding and blocking experiments is illustrated in Figure 15 along with the prior versions of the CDR-grafted heavy chain. Clearly the changes made at residues 42 and 44 in previous versions were not necessary, contrary to the criteria set forth in PCT/US89/05857. The change at residue 60, present in HCDR10, but not in HCDR9, is beneficial.

A summary of the CDR-grafted OKT4A heavy chains and their activities in the binding and blocking assays is shown in Table 1. The most active CDR-grafted OKT4A antibody which contains the fewest murine residues is the combination of HCDR10 and LCDR2.

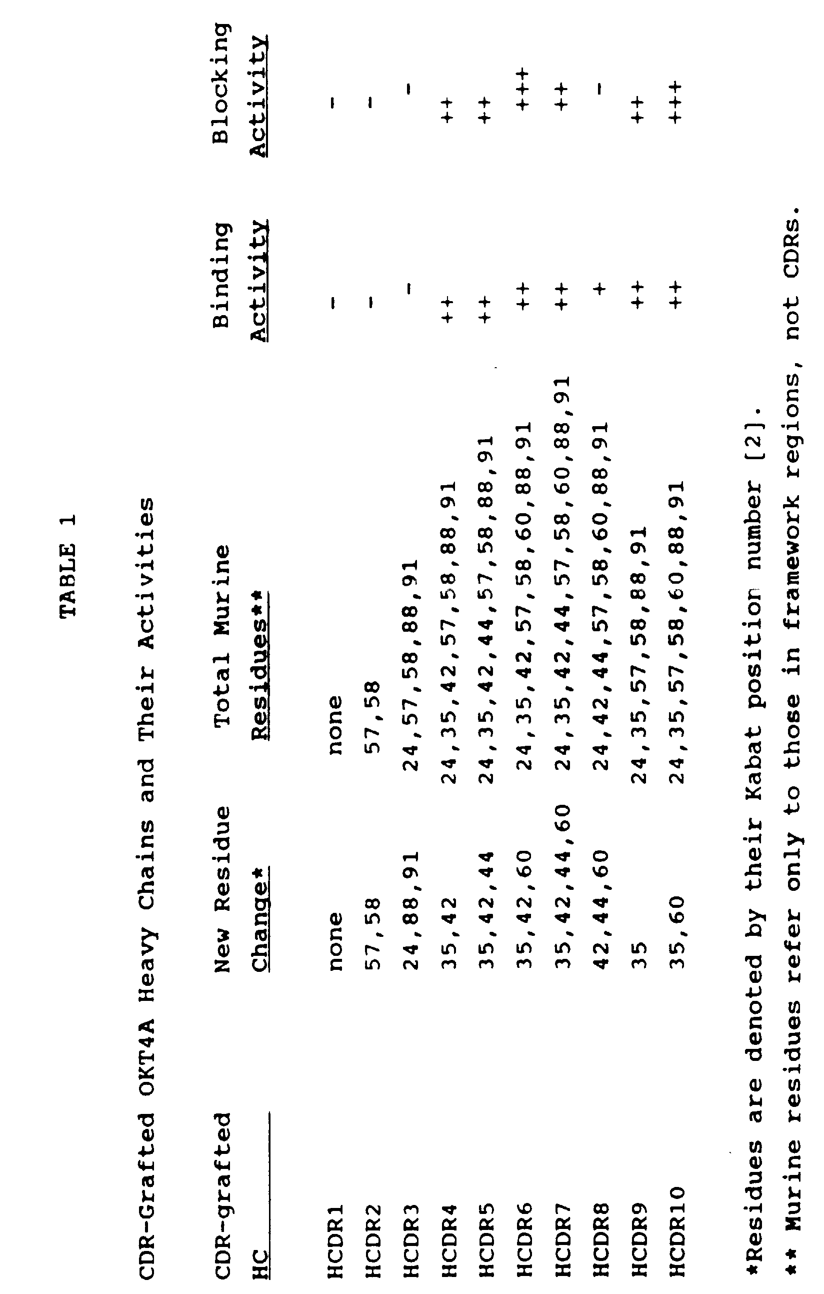

### Alternative Light Chain Constructs

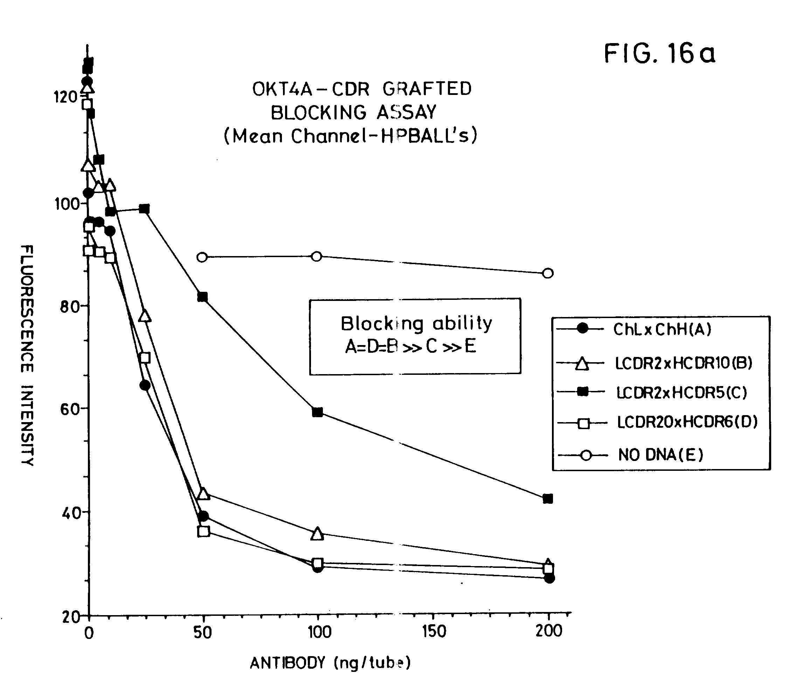

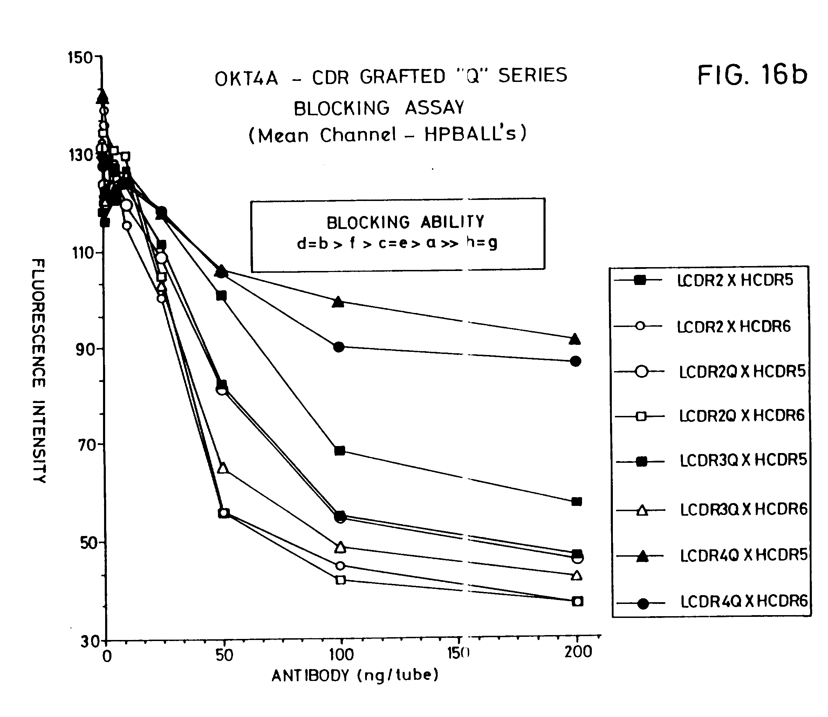

As is stated above, the present light chain constructs were produced on the assumption that at position 27 in the OKT4A light chain, there was a proline residue. Once it was appreciated that position 27 should be a glutamine residue, three new light chain constructs were produced and expressed. These were labelled LCDR2Q, LCDR3Q and LCDR4Q are identical to LCDR2, LCDR3 and LCDR4 respectively except that at position 27 there is a glutamine (Q) instead of a proline (P) residue. It has been shown that these light chains retained full activity. The data showing this is presented in Figure 16.

It is to be noted that proline is significantly different from all other amino acids in that it has a planar structure. It is therefore commonly found at sites in peptide sequences where a change in orientation of the chain occurs. It is therefore likely that the structure of the light chain CDR1 having proline at residue 27 will be significantly different from that of the light chain CDR1 having glutamine at residue 27. Despite this, it has been demonstrated that the two light chains are equivalent from a functional standpoint. This supports the view expressed herein that it is not necessary to change all 6 CDRs in an antibody in order to produce a functional CDR-grafted antibody.

### Alternative Modifications of the CDR-Grafted Light and Heavy Chains

Residue changes made in later versions of the CDR-grafted light and heavy chains were done based upon molecular modelling of REI, KOL and a related mouse antibody, MOPC 603, rather than of the CDR-grafted antibodies themselves. Some of the alterations may be unnecessary for binding, especially at lower binding affinities. We have constructed several CDR-grafted light and heavy chain genes in which some of the framework residues previously switched to mouse residues have been changed back to the human. Generally those residues not directly involved in lengthening CDRs or positioning CDRs are being changed back to the human residues in various combinations. Table 2 lists these light and heavy chain genes with the residue numbers that revert from the murine to human. Site-directed mutagenesis was used to construct these genes. They will be expressed in COS cells and their ability to recognize CD4 will be tested in the binding and blocking assays. The most desirable CDR-grafted antibody is the one with the fewest murine residues that is capable of recognizing CD4 with an affinity similar to that of murine OKT4A.

**TABLE 2**

| MODIFICATIONS TO THE CDR-GRAFTED LIGHT AND HEAVY CHAINS | | |
|---|---|---|
| Construct | Residue Change* | Total murine residues** |
| Light chain: | | |
| LCDR3 | 38 | 33,34,49,89 |
| LCDR4 | 49 | 33,34,38,89 |
| LCDR5 | 89 | 33,34,38,89 |
| LCDR6 | 38,49,89 | 33,34 |
| | | |

| Heavy chain: | | |
|---|---|---|
| HCDR11 | 88,91 | 24,35,47,58,60 |
| HCDR12 | 24,88,91 | 35,57,58,60 |

| | | |
|---|---|---|
| *Residues are denoted by their Kabat position number [2]. Noted residues will be changed from murine sequence to human sequence. | | |
| ** Murine residues refer to residues in frameworks, not CDRs. | | |

### Determination of Relative Binding Affinity

The relative binding affinities of CDR-grafted anti-CD4 monoclonal antibodies were determined by competition binding [8] using the HPB-ALL human T cell line as a source of CD4 antigen and fluorescein-conjugated murine OKT4A (F1-OKT4A) of known binding affinity as a tracer antibody. The binding affinity of F1-OKT4A tracer antibody was determined by a direct binding assay in which increasing amount of F1-OKT4A were incubated with HPB-ALL (5 x 10⁵) in PBS with 5% fetal calf serum for 60 min at 40°C. Cells were washed, and the fluorescence intensity was determined on a FACScan flow cytometer calibrated with quantative microbead stands (Flow Cytometry Standards, Research Triangle Park, NC). Florescence intensity per antibody molecule (F/P ratio) was determined by using microbeads which have a predetermined number of mouse IgG antibody binding sites (Simply Cellular Beads, Flow Cytometry Standards). F/P equals the florescence intensity of beads saturated with F1-OKT4A divided by the number of binding sites per beads. The amount of bound and free F1-OKT4A was calculated from the mean fluorescence intensity per cell, and the ratio of bound/free was plotted against the number of moles of antibody bound. A linear fit was used to determine the affinity of binding (absolute value of the slope).

For competitive binding, increasing amounts of competitor antibody were added to a sub-saturating dose of F1-OKT4A and incubated with 5 x 10⁵ HPB-ALL in 200 µl of PBS with 5% fetal calf serum for 60 min at 4°C. The fluorescence intensities of the cells were measured on a FACScan flow cytometer calibrated with quantitative microbead standards. The concentrations of bound and free F1-OKT4A were calculated. The affinities of competing antibodies were calculated from the equation [X] -[OKT4A] = (1/Kx) - (1/Ka), where Ka is the affinity of muring OKT4A, Kx is the affinity of competitor X [ ] is the concentration of competitor antibody at which bound/free binding is R/2, and R is the maximal bound/free binding.

### Affinity Results

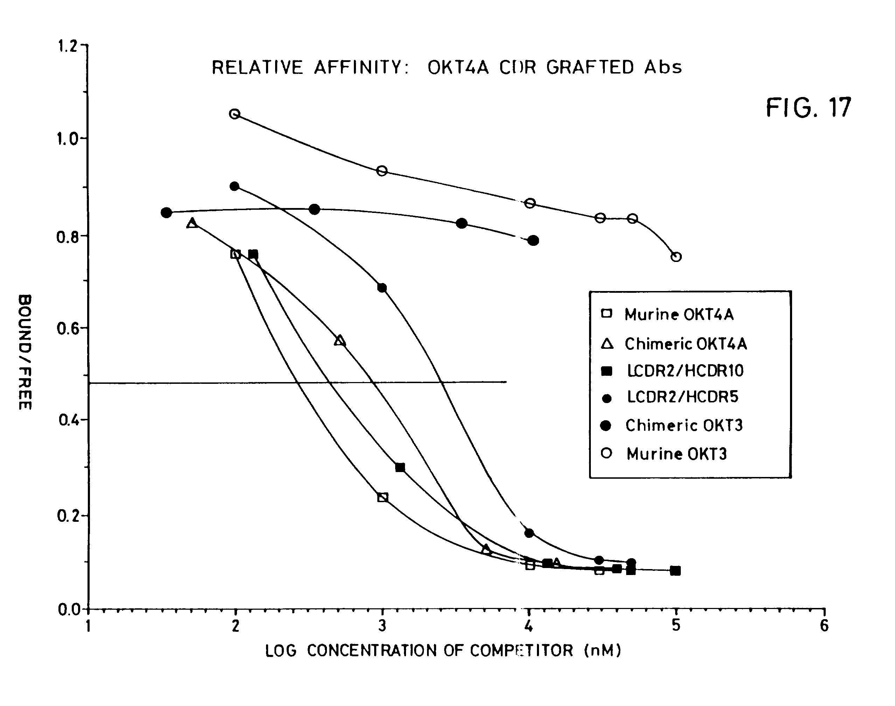

The relative affinity constants of the humanized antibodies (Fig. 17, Table 3) were determined, and LCDR2 combined with HCDR10 retained 68% of the activity of the parent. LCDR2/HCDR5 (Table 1) retained only 13% of the murine antibody affinity. These results are in agreement with those obtained in blocking assays (Fig. 16a&b). Comparison of HCDR5 with HCDR7 (Fig. 15) suggests that residue 60, while not critical for activity, is beneficial when converted to that in the donor sequence. In the same figure, the deleterious effect of the donor residue at position 44 can also be seen (HCDR4 vs. HCDR5).

**TABLE 3**

| Relative Affinity Constants of the CDR-grafted Antibodies | | | |
|---|---|---|---|
| Antibody Constructs | Log conc. competitor (pM) at 50% inhibition | | Affinity Constant (Kx) |
| Murine OKT4A | 2.4 | | 3 X 10⁹ |
| Chimeric OKT4A | 2.9 | | 1.1 x 10⁹ |
| LCDR2/HCDR10 | 2.6 | | 2.1 x 10⁹ |
| LCDR2/HCDR5 | 3.4 | | 0.4 x 10⁹ |
| Chimeric OKT3 | | No inhibition | |
| Murine OKT3 | | No inhibition | |

### Functional Studies

It is believed that the CD4 antigen, which is recognised by OKT4A and its chimeric and CDR-grafted equivalents, is involved in the interactions which give rise to the biological functions of T lymphocytes carrying the CD4 antigen. In particular, it is believed that the CD4 antigen is involved in the mixed lymphocyte reaction (MLR) and in the proliferation of peripheral blood mononuclear cells (PBMC). In order to show that the CDR-grafted antibodies of the present invention are likely to have the same biological activity as murine OKT4A, the following functional studies were carried out.

### Inhibition of MLR

Human PBMC were isolated by density gradient centrifugation with Ficoll® and resuspended in complete DMEM containing 1% foetal calf serum (FCS). 2 x 10⁵ responder PBMC and 1 x 10⁵ irradiated (2 Mrad) allogeneic PBMC were added to each well of a 96 well tissue culture plate, followed by serial dilutions of a purified anti-CD4 antibody. Cells were cultured for 6 days, pulsed with ³H thymidine for 24 hours and harvested. ³H-thymidine incorporation was measured by liquid scintillation.

As a negative control, irradiated responder cells were used in place of the irradiated allogeneic PBMC and no antibody was added. As a positive control, the experiment was carried out without the addition of antibody. In the experiment, the antibodies used were murine OKT4A, chimeric OKT4A and the F(ab')₂ fragment of murine OKT4A.

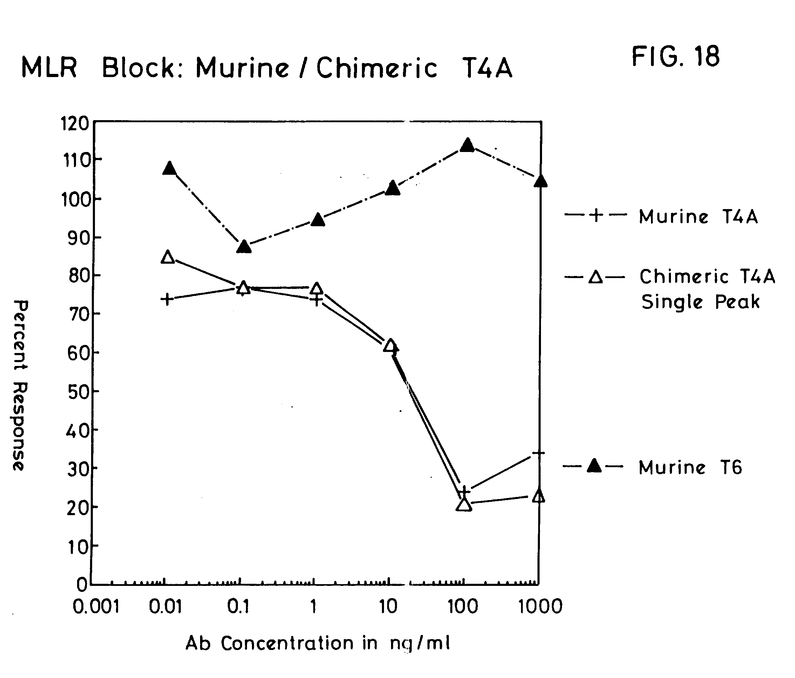

The results of the experiment are shown in Figure 18. Both the chimeric OKT4A and the murine OKT4A showed similar inhibition of MLR.

### Inhibition of Proliferation

OKT3 (20 ng/ml), a murine MAb which recognises the CD3 antigen on T lymphocytes, was immobilised on polystyrene 96 well tissue culture plates for 4 hours at 20°C. The plates were washed three times with phosphate buffered saline (PBS) and 1 x 10⁵ PMBC were added to each well. Thereafter, serial dilutions of an anti-CD4 antibody were added. Cells were cultured for 72 hours, pulsed with ³H-thymidine for 24 hours and harvested. ³H-thymidine incorporation was measured by liquid scintillation.

As a negative control, proliferation was measured in the absence of both the OKT3 and anti-CD4 antibodies. As a positive control proliferation was measured in the presence of OKT3 alone. In this experiment, the antibodies used were murine OKT4A, chimeric OKT4A and the F(ab')₂ fragment of murine OKT4A.

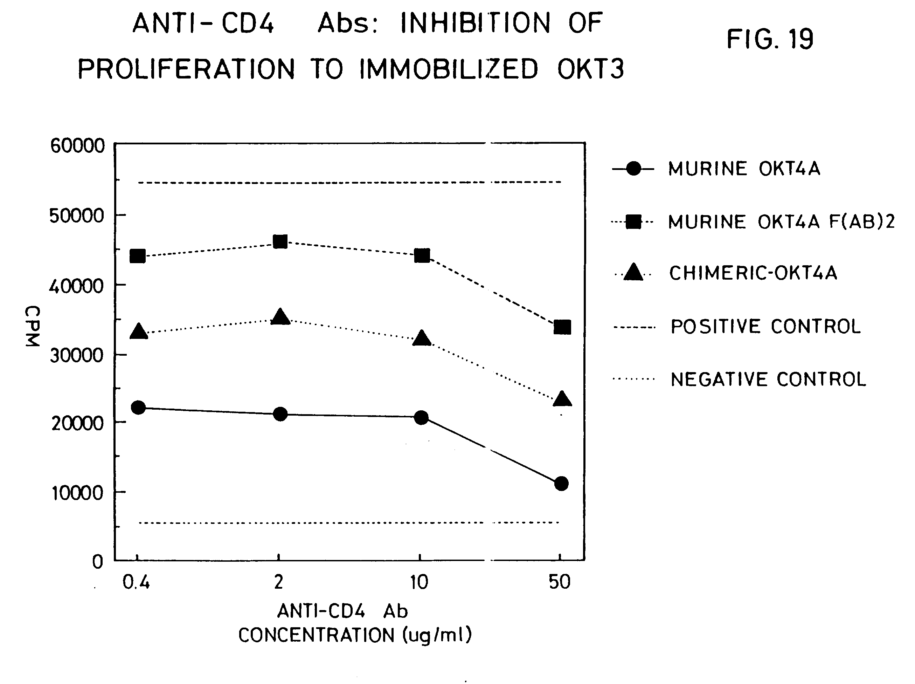

The results are given in Figure 19, which shows that chimeric OKT4A has substantially the same ability to inhibit proliferation as does murine OKT4A.

The above functional studies show that chimeric OKT4A has equivalent biological properties to murine OKT4A. Since the CDR-grafted anti-CD4 antibodies have substantially the same affinity for the CD4 antigen as the chimeric OKT4A antibody and since the chimeric OKT4A antibody has the same constant domains as the CDR-grafted OKT4A antibodies, it can be expected that the CDR-grafted OKT4A antibodies will have the same biological functions as murine OKT4A and will thus be of use in therapy.

### SUMMARY

A number of different CDR-grafted OKT4A antibodies have been generated. Essentially, DNA encoding the CDRs of the murine OKT4A heavy and light chains has been grafted onto the frameworks of the human heavy chain KOL and light chain REI antibody genes. These variable domains are ligated to the DNA encoding human kappa light chain and IgG4 heavy chain constant portion. The resulting CDR-grafted genes are expressed in COS-1 cells. Antibody secreted into the tissue culture media is collected, quantified by ELISA, and tested for its ability to bind to CD4 positive cells and to block the binding of murine OKT4A.

The initially designed CDR-grafted antibody was unable to interact with CD4. A number of modifications were made to the light chain where critical human framework residues in the REI sequence, identified by molecular modelling, were changed to the murine OKT4A residues. This new version of the light chain, LCDR2, was able to recognize the CD4 antigen. Similarly, a number of heavy chain human framework residues were changed to murine in various combinations to generate HCDR2 through HCDR10. Several of these heavy chains, in combination with LCDR2, competed well with the murine OKT4A antibody for CD4. Presently the CDR-grafted OKT4A of choice is the combination of LCDR2Q and HCDR10. Further versions of the light and heavy chains are currently being generated where framework residues that were previously switched to the murine residues are being changed back to human. These more humanized CDR-grafted antibodies will be tested for their ability to recognize CD4.

### REFERENCES

[1] Kabat et al., in Sequences of Proteins of Immunological Interest, US Department of Health and Human Services, 1987.
[2] Wu and Kabat, J. Exp. Med., 132, 211, 1970.
[3] Oi et al., PNAS-USA, 80, 825, 1983.
[4] Neuberger, EMBO J., 2, 1373, 1983.
[5] Ochi et al., PNAS-USA, 80, 6351, 1983.
[6] Verhoeyen et al., Science, 239, 1534, 1988.
[7] Reichmann et al., Nature, 332, 323, 1988.
[8] Queen et al., PNAS-USA, 86, 10029, 1989.
[9] Zoller and Smith, Nuc. Acids Res., 10, 6487, 1982.
[10] Norris et al., Nuc. Acids Res., 11, 5103, 1983.
[11] Zoller and Smith, DNA, 3, 479, 1984.
[12] Kramer et al., Nuc. Acids Res., 10, 6475, 1982.
[13] Mullis and Foloona, Meth. Enz., 155, 335, 1987.
[14] Higuchi et al., Nuc. Acids Res., 16, 7351, 1988.
[15] Ho et al., Gene, 77, 51, 1989.
[16] Ho et al., in Engineering Hybridisation Restriction Genes without the Use of Restriction Enzymes: Gene Splicing by Overlap Extension.
[17] Horton et al., Gene, 77, 61, 1989.
[18] Bolivar et al., Gene, 2, 95, 1977.
[19] Chang et al., Nature, 275, 615, 1978.
[20] Itakura et al., Science, 198, 1056, 1978.
[21] Goedell et al., Nature, 281, 544, 1979.
[22] Goedell et al., Nuc. Acids Res., 8, 4057, 1980.
[23] Siebenlist et al., Cell, 20, 269, 1980.
[24] Stinchcomb et al., Nature, 282, 39, 1979.
[25] Kingsman et al., Gene, 7, 141, 1979.
[26] Tschemper et al., Gene, 19, 157, 1980.
[27] Jones, Genetics, 85, 12, 1977.
[28] Hitzeman et al., J. Biol. Chem., 255, 2073, 1980.
[29] Hess et al., J. Adv. Enzyme Reg., 7, 149, 1968.
[30] Holland et al., Biochemistry, 17, 4900, 1978.
[31] Kruse and Patterson, in Tissue Culture, Academic Press, 1973.
[32] Fiers et al., Nature, 273, 113, 1978.
[33] Maniatis et al., in Molecular Cloning: A Laboratory Manual, 2nd Edition, Cold Spring Harbor Press, 1990.
[34] Scopes, in Protein Purification, Springer Verlag, NY, 1982.
[35] Lefkovite and Pernis, in Immunological Methods, Volumes I and II, Academic Press, NY, 1979 and 1981.
[36] Bernhard et al., in Leukocyte Typing, Springer Verlag, NY, 1984.
[37] Mack, in Remington's Pharmaceutical Sciences, 16th Edition, 1982.
[38] Chirgwin et al., Biochemistry, 18, 5294, 1979.
[39] Sanger et al., PNAS-USA, 74, 5463, 1977.
[40] Jones et al., Nature, 321, 522, 1986.
[41] Whittle et al., Protein Engineering, 1, 499, 1987.
[42] Kozak et al., J. Mol. Biol., 196, 947, 1987.
[43] Birnboim and Doly, Nuc. Acids Res., 14, 1513, 1979.
[44] Gluzman, Cell, 23, 175, 1981.
[45] Lopata et al., Nuc. Acids Res., 14, 5707, 1984.
[46] Kunkel, PNAS-USA, 82, 488, 1985.
[47] Chotia and Lesk, J. Mol. Biol., 96, 4, 901, 1987.
[48] Baranov et al., Gene, 84, 2, 463, 1989.

## Claims

1. An antibody molecule capable of binding to the CD4 antigen comprising a composite heavy chain and a complementary light chain wherein, in the variable domain of said composite heavy chain, the framework regions are predominantly derived from a human antibody (acceptor) and at least residues 23, 24, 26 to 35, 49 to 65 and 95 to 102 (according to the Kabat numbering system) correspond to the equivalent residues in the mouse monoclonal antibody OKT4A (donor) as shown in Figure 3 of the accompanying drawings.

2. The antibody molecule of claim 1, wherein residues 6 and 48 in the composite heavy chain additionally correspond to the equivalent residues in the donor antibody.

3. The antibody molecule of claim 1 or claim 2, wherein residues 71, 73 and 79 in the composite heavy chain additionally correspond to the equivalent residues in the donor antibody.

4. The antibody molecule of any one of claims 1 to 3, wherein any one or any combination of residues 57, 58, 60, 88 and 91 in the composite heavy chain correspond to the equivalent residues in the donor antibody.

5. The antibody molecule of any one of claims 1 to 4, wherein the acceptor residues in the composite heavy chain correspond to the equivalent residues in the human KOL heavy chain as shown in Figure 5 of the accompanying drawings.

6. The antibody molecule of any one of claims 1 to 5, wherein the complementary light chain is a composite light chain wherein, in the variable domain of said composite light chain, the framework regions are predominantly derived from a first antibody (acceptor) and at least residues 24 to 34, 49 to 56 and 89 to 97 (according to the Kabat numbering system) correspond to the equivalent residues in the mouse monoclonal antibody OKT4A (donor) as shown in Figure 4 of the accompanying drawings.

7. An antibody molecule capable of binding to the CD4 antigen comprising a composite light chain and a complementary heavy chainwherein, in the variable domain of said composite light chain, the framework regions are predominantly derived from a human antibody (acceptor) and at least residues 24 to 34, 49 to 56 and 89 to 97 (according to the Kabat numbering system) correspond to the equivalent residues in the mouse monoclonal antibody OKT4A (donor) as shown in Figure 4 of the accompanying drawings.

8. The antibody molecule of claim 6 or claim 7, wherein residue 89 in the composite light chain additionally corresponds to the equivalent residue in the donor antibody.

9. The antibody molecule of any one of claims 6 to 8, wherein the acceptor residues in the composite light chain correspond to the equivalent residues in the human REI light chain as shown in Figure 6 of the accompanying drawings.

10. The antibody molecule of any one of claims 1 to 9, which has an affinity for the CD4 antigen of from 10⁵.M⁻¹ to 10¹².M⁻¹.

11. The antibody molecule of claim 10, which has an affinity for the CD4 antigen of at least about 10⁸.M⁻¹.

12. The antibody molecule of claim 10 or claim 11, which has an affinity for the CD4 antigen similar to that of OKT4A.

13. The antibody molecule of any one of claims 1 to 12, which is a complete Ig.

14. The antibody molecule of claim 13, which is of isotype IgG₄.

15. The antibody molecule of claim 13 or claim 14, wherein one or more residues in the constant domains of the Ig has been altered in order to alter the effector functions of the constant domains.

16. The antibody molecule of any one of claims 1 to 15 which is produced by use of recombinant DNA technology.

17. A method for producing an antibody molecule according to any one of claims 1 to 16, which method comprises: providing a first DNA sequence, encoding a composite heavy chain as defined in any one of claims 1 to 5 or a composite light chain as defined in any one of claims 6 to 9, under the control of suitable upstream and downstream elements; transforming a host cell with the first DNA sequence; and culturing the transformed host cell so that an antibody molecule according to any one of claims 1 to 18 is produced.

18. The method of claim 17, which further comprises: providing a second DNA sequence, encoding an antibody light or heavy chain complementary to the first chain, under the control of suitable upstream and downstream elements; and transforming the host cell with both the first and second DNA sequences.

19. The method of claim 18, wherein the second DNA sequence encodes a composite antibody chain.

20. The method of claim 18 or claim 19, wherein the first and second DNA sequences are present on the same vector.

21. The method of claim 20, wherein the sequences are under the control of the same upstream and/or downstream elements.

22. The method of claim 20, wherein the sequences are under the control of different upstream and/or downstream elements.

23. The method of claim 18 or claim 19, wherein the first and second DNA sequences are present on different vectors.

24. The method of any one of claims 17 to 23, wherein the host cell is a CHO cell.

25. A nucleotide sequence which encodes a composite antibody chain as defined in any one of claims 1 to 9.

26. The antibody molecule of any one of claims 1 to 16, for use in therapy, in particular in treating graft rejections or in treating helper T cell disorders.

27. A pharmaceutical composition comprising the antibody molecule of any one of claims 1 to 16 in combination with a pharmaceutically acceptable excipient.

## Patentansprüche

1. Anitkörpermolekül, befähigt zur Bindung an das CD4-Antigen, umfassend eine schwere Verbundkette und eine komplementäre leichte Kette, wobei in der variablen Domäne der schweren Verbundkette die Gerüstregionen vorwiegend von einem humanen Antikörper (Akzeptor) abgeleitet sind und mindestens Reste 23, 24, 26 bis 35, 49 bis 65 und 95 bis 102 (entsprechend dem Kabat-Numerierungssystem) den äquivalenten Resten im monoklonalen Mäuse-Antikörper OKT4A (Donator) gemäß der Darstellung in Fig. 3 der beigefügten Zeichnung entsprechen.

2. Antikörpermolekül nach Anspruch 1, wobei die Reste 6 und 48 in der schweren Verbundkette zusätzlich den äquivalenten Resten im Donator-Antikörper entsprechen.

3. Antikörpermolekül nach Anspruch 1 oder 2, wobei die Reste 71, 73 und 79 in der schweren Verbundkette zusätzlich den äquivalenten Resten im Donator-Antikörper entsprechen.

4. Antikörpermolekül nach einem der Ansprüche 1 bis 3, wobei einer der Reste 57, 58, 60, 88 und 91 oder eine beliebige Kombination davon in der schweren Verbundkette den äquivalenten Resten im Donator-Antikörper entsprechen.

5. Antikörpermolekül nach einem der Ansprüche 1 bis 4, wobei die Akzeptorreste in der schweren Verbundkette den äquivalenten Resten in der humanen schweren KOL-Kette gemäß Fig. 5 der beigefügten Zeichnung entsprechen.

6. Antikörpermolekül nach einem der Ansprüche 1 bis 5, wobei es sich bei der komplementären leichten Kette um eine leichte Verbundkette handelt, wobei die Gerüstregionen in der variablen Domäne der leichten Verbundkette sich vorwiegend von einem ersten Antikörper (Akzeptor) ableiten und mindestens Reste 24 bis 34, 49 bis 56 und 89 bis 97 (entsprechend dem Kabat-Numerierungssystem) den äquivalenten Resten im monoklonalen Mäuse-Antikörper OKT4A (Donator) gemäß der Darstellung in Fig. 4 der beigefügten Zeichnung entsprechen.

7. Antikörpermolekül, das zur Bindung an das CD4-Antigen befähigt ist, umfassend eine leichte Verbundkette und eine komplementäre schwere Kette, wobei in der variablen Domäne der leichten Verbundkette die Gerüstbereiche vorwiegend von einem humanen Antikörper (Akzeptor) abgeleitet sind und mindestens Reste 24 bis 34, 49 bis 56 und 89 bis 97 (entsprechend dem Kabat-Numerierungssystem) den äquivalenten Resten im monoklonalen Mäuse-Antikörper OKT4A (Donator) gemäß der Darstellung in Fig. 4 der beigefügten Zeichnung entsprechen.

8. Antikörpermolekül nach Anspruch 6 oder 7, wobei der Rest 89 in der leichten Verbundkette zusätzlich dem äquivalenten Rest im Donator-Antikörper entspricht.

9. Antikörpermolekül nach einem der Ansprüche 6 bis 8, wobei die Akzeptorreste in der leichten Verbundkette den äquivalenten Resten in der humanen leichten REI-Kette gemäß der Darstellung in Fig. 6 der beigefügten Zeichnung entsprechen.

10. Antikörpermolekül nach einem der Ansprüche 1 bis 9, das eine Affinität für das CD4-Antigen von 10⁵·M⁻¹ bis 10¹²·M⁻¹ aufweist.

11. Antikörpermolekül nach Anspruch 10, das eine Affinität für das CD4-Antigen von mindestens etwa 10⁸·M⁻¹ aufweist.

12. Antikörpermolekül nach Anspruch 10 oder 11, das eine Affinität für das CD4-Antigen ähnlich der von OKT4A aufweist.

13. Antikörpermolekül nach einem der Ansprüche 1 bis 12, wobei es sich um vollständiges Ig handelt.

14. Antikörpermolekül nach Anspruch 13, das vom Isotyp IgG₄ ist.

15. Antikörpermolekül nach Anspruch 13 oder 14, wobei einer oder mehrere Reste in den konstanten Domänen des Ig verändert worden sind, um die Effektorfunktionen der konstanten Domänen zu verändern.

16. Antikörpermolekül nach einem der Ansprüche 1 bis 15, hergestellt unter Anwendung von rekombinanter DNA-Technik.

17. Verfahren zur Herstellung eines Antikörpermoleküls nach einem der Ansprüche 1 bis 16, wobei das Verfahren folgendes umfaßt:
das Bereitstellen einer ersten DNA-Sequenz, die für eine schwere Verbundkette gemäß der Definition in einem der Ansprüche 1 bis 5 oder eine leichte Verbundkette gemäß der Definition in einem der Ansprüche 6 bis 9 kodiert, unter der Kontrolle von geeigneten stromaufwärtigen und stromabwärtigen Elementen; das Transformieren einer Wirtszelle mit der ersten DNA-Sequenz; und das Züchten der transformierten Wirtszelle, so daß ein Antikörpermolekül nach einem der Ansprüche 1 bis 16 gebildet wird.

18. Verfahren nach Anspruch 17, ferner umfassend:
das Bereitstellen einer zweiten DNA-Sequenz, die für eine mit der ersten Kette komplementäre leichte oder schwere Antikörper-Kette kodiert, unter der Kontrolle von geeigneten stromaufwärtigen und stromabwärtigen Elementen; und das Transformieren der Wirtszelle mit der ersten und zweiten DNA-Sequenz.

19. Verfahren nach Anspruch 18, wobei die zweite DNA-Sequenz für eine Antikörper-Verbundkette kodiert.

20. Verfahren nach Anspruch 18 oder 19, wobei die erste und die zweite DNA-Sequenz am gleichen Vektor vorhanden sind.

21. Verfahren nach Anspruch 20, wobei die Sequenzen unter der Kontrolle der gleichen stromaufwärtigen und/oder stromabwärtigen Elemente stehen.

22. Verfahren nach Anspruch 20, wobei die Sequenzen unter der Kontrolle von unterschiedlichen stromaufwärtigen und/oder stromabwärtigen Elementen stehen.

23. Verfahren nach Anspruch 18 oder 19, wobei die erste und zweite DNA-Sequenz an unterschiedlichen Vektoren vorhanden sind.

24. Verfahren nach einem der Ansprüche 17 bis 23, wobei es sich bei der Wirtszelle um eine CHO-Zelle handelt.

25. Nucleotidsequenz, die für eine Antikörper-Verbundkette gemäß der Definition in einem der Ansprüche 1 bis 9 kodiert.

26. Antikörpermolekül nach einem der Ansprüche 1 Dis ib zur Verwendung in der Therapie, insbesondere zur Behandlung von Transplantatabstoßungen oder zur Behandlung von Störungen von Helfer T-Zellen.

27. Pharmazeutische Zusammensetzung, enthaltend das Antikörpermolekül nach einem der Ansprüche 1 bis 16 in Kombination mit einem pharmazeutisch verträglichen Träger.

## Revendications

1. Molécule d'anticorps capables de se lier à l'antigène CD4 comportant une chaîne lourde composite et une chaîne légère complémentaire dans laquelle, dans le domaine variable de ladite chaîne lourde composite, les régions du squelette de base sont dérivées de manière prédominante à partir d'un anticorps humain (receveur) et au moins les résidus 23, 24, 26 à 35, 49 à 65 et 95 à 102 (selon le système de numérotation de Kabat) correspondent aux résidus équivalents situés dans l'anticorps OKT4A monoclonal de souris (donneur) comme représenté sur la figure 3 des dessins annexés.

2. Molécule d'anticorps selon la revendication 1, dans laquelle les résidus 6 et 48 situés dans la chaîne lourde composite correspondent de plus aux résidus équivalents situés dans l'anticorps donneur.

3. Molécule d'anticorps selon la revendication 1 ou 2, dans laquelle les résidus 71, 73 et 79 situés dans la chaîne lourde composite correspondent de plus aux résidus équivalents situés dans l'anticorps donneur.

4. Molécule d'anticorps selon l'une quelconque des revendications 1 à 3, dans laquelle tout résidu ou toute combinaison des résidus 57, 58, 60, 88 et 91 situés dans la chaîne lourde composite correspond aux résidus équivalents situés dans l'anticorps donneur.

5. Molécule d'anticorps selon l'une quelconque des revendications 1 à 4, dans laquelle les résidus du receveur situés dans la chaîne lourde composite correspondent aux résidus équivalents situés dans la chaîne lourde de KOL humain comme représenté sur la figure 5 des dessins annexés.

6. Molécule d'anticorps selon l'une quelconque des revendications 1 à 5, dans laquelle la chaîne légère complémentaire est une chaîne légère composite dans laquelle, dans le domaine variable de ladite chaîne légère composite, les régions du squelette de base sont dérivées de manière prédominante à partir d'un premier anticorps (receveur) et au moins les résidus 24 à 34, 49 à 56 et 89 à 97 (selon le système de numérotation de Kabat) correspondent aux résidus équivalents situés dans l'anticorps OKT4A monoclonal de souris (donneur) comme représenté sur la figure 4 des dessins annexés.

7. Molécule d'anticorps capable de se lier à l'antigène CD4 comportant une chaîne légère composite et une chaîne lourde complémentaire dans laquelle, dans le domaine variable de ladite chaîne légère composite, les régions du squelette de base sont dérivées de manière prédominante à partir d'un anticorps humain (receveur) et au moins les résidus 24 à 34, 49 à 56 et 89 à 97 (selon le système de numérotation de Kabat) correspondent aux résidus équivalents situés dans l'anticorps OKT4A monoclonal de souris (donneur) comme représenté sur la figure 4 des dessins annexés.

8. Molécule d'anticorps selon la revendication 6 ou 7, dans laquelle le résidu 89 situé dans la chaîne légère composite correspond de plus au résidu équivalent situé dans l'anticorps du donneur.

9. Molécule d'anticorps selon l'une quelconque des revendications 6 à 8, dans laquelle les résidus du receveur situés dans la chaîne légère composite correspondent aux résidus équivalents situés dans la chaîne légère de REI humain comme représenté sur la figure 6 des dessins annexés.

10. Molécule d'anticorps selon l'une quelconque des revendications 1 à 9, qui a une affinité pour l'antigène CD4 allant de 10⁵ . M⁻¹ à 10¹² . M⁻¹.

11. Molécule d'anticorps selon la revendication 10, qui a une affinité pour l'antigène CD4 d'au moins environ 10⁸. M⁻¹.

12. Molécule d'anticorps selon la revendication 10 ou 11, qui a une affinité pour l'antigène CD4 similaire à celle de OKT4A.

13. Molécule d'anticorps selon l'une quelconque des revendications 1 à 12 qui est une Ig entière.

14. Molécule d'anticorps selon la revendication 13, qui est de l'isotype IgG₄.

15. Molécule d'anticorps selon la revendication 13 ou 14, dans laquelle un ou plusieurs résidus situés dans les domaines constants de l'Ig a été modifié ou ont été modifiés dans le but de modifier les fonctions effectrices des domaines constants.

16. Molécule d'anticorps selon l'une quelconque des revendications 1 à 15, qui est produite en utilisant la technologie de l'ADN recombinant.

17. Procédé pour produire une molécule d'anticorps selon l'une quelconque des revendications 1 à 16, lequel procédé comporte les étapes consistant à :
fournir une première séquence d'ADN, codant pour une chaîne lourde composite telle que définie selon l'une quelconque des revendications 1 à 5 ou une chaîne légère composite telle que définie selon l'une quelconque des revendications 6 à 9, sous la commande d'éléments adaptés situés en amont et en aval ; transformer une cellule hôte à l'aide de la première séquence d'ADN ; et cultiver la cellule hôte transformée de sorte qu'une molécule d'anticorps selon l'une quelconque des revendications 1 à 18 soit produite.

18. Procédé selon la revendication 17, qui comporte de plus les étapes consistant à:
fournir une seconde séquence d'ADN, codant pour une chaîne légère ou lourde d'anticorps complémentaire à la première chaîne, sous la commande d'éléments adaptés situés en amont et en aval ; et transformer la cellule hôte à l'aide à la fois de la première et de la seconde séquence d'ADN.

19. Procédé selon la revendication 18, dans lequel la seconde séquence d'ADN code pour une chaîne composite d'anticorps.

20. Procédé selon la revendication 18 ou 19, dans lequel les première et seconde séquences sont présentes sur le même vecteur.

21. Procédé selon la revendication 20, dans lequel les séquences sont sous la commande des mêmes éléments situés en amont et/ou en aval.

22. Procédé selon la revendication 20, dans lequel les séquences sont sous la commande d'éléments différents situés en amont et/ou en aval.

23. Procédé selon la revendication 18 ou 19, dans lequel les première et seconde séquences d'ADN sont présentes sur différents vecteurs.

24. Procédé selon l'une quelconque des revendications 17 à 23, dans lequel la cellule hôte est une cellule CHO.

25. Séquence nucléotidique qui code pour une chaîne composite d'anticorps telle que définie selon l'une quelconque des revendications 1 à 9.

26. Molécule d'anticorps selon l'une quelconque des revendications 1 à 16, destinée à une utilisation en thérapie, en particulier dans le traitement des rejets de greffe ou dans le taitement concernant les troubles de cellules T auxiliaires.

27. Composition pharmaceutique comportant la molécule d'anticorps selon l'une quelconque des revendications 1 à 16 en combinaison avec un excipient pharmaceutiquement acceptable.
